# EUROPEAN PATENT SPECIFICATION

(11) **EP 1 313 762 B1**
(45) Date of publication and mention of the grant of the patent: **05.07.2006**
(21) Application number: 01954168.9
(22) Date of filing: 06.08.2001
(51) Int. Cl.: C07K 14/47, C07K 16/18, C12N 15/11, C12N 15/12, C12N 5/10, C12Q 1/68, A61K 38/17, A61K 48/00, G01N 33/50, G01N 33/53

(54) **SUPPRESSOR GENE**
SUPPRESSOR-GEN
GENES SUPPRESSEURS

(30) Priority: 04.08.2000 GB 0019018; 08.12.2000 GB 0029996; 26.05.2001 GB 0112890
(43) Date of publication of application: 28.05.2003
(73) Proprietor: LUDWIG INSTITUTE FOR CANCER RESEARCH, 8024 Zürich (CH)
(72) Inventor: LU, Xin, Ludwig Inst. for Cancer Research, London W2 1PG (GB)
(74) Representative: Harrison Goddard Foote
(86) International application number: PCT/GB2001/003524
(87) International publication number: WO 2002/012325

(56) References cited:
- WO-A-00/20587
- WO-A-00/32628
- WO-A-01/53312
- WO-A-01/57190
- WO-A-99/15657
- YANG JIAN-PING ET AL: "NF-kappaB subunit p65 binds to 53BP2 and inhibits cell death induced by 53BP2." ONCOGENE, vol. 18, no. 37, pages 5177-5186, XP001027267 ISSN: 0950-9232
- IWABUCHI KUNIYOSHI ET AL: "Stimulation of p53-mediated transcriptional activation by the p53-binding proteins, 53BP1 and 53BP2." JOURNAL OF BIOLOGICAL CHEMISTRY, vol. 273, no. 40, 2 October 1998 (1998-10-02), pages 26061-26068, XP002189291 ISSN: 0021-9258
- NAUMOVSKI L & CLEARY M L: "The p53-binding protein 53BP2 also interacts with Bcl2 and impedes cell cycle progression at G2/M" MOLECULAR BIOLOGY OF THE CELL, BETHESDA, MD, US, vol. 16, no. 7, 1 July 1996 (1996-07-01), pages 3884-3892, XP002095578 ISSN: 1059-1524 cited in the application
- NAGASE T ET AL: "PREDICTION OF THE CODING SEQUENCES OF UNIDENTIFIED HUMAN GENES. XI. THE COMPLETE SEQUENCES OF 100 NEW CDNA CLONES FROM BRAIN WHICH CODE FOR LARGE PROTEINS IN VITRO" DNA RESEARCH, UNIVERSAL ACADEMY PRESS, JP, vol. 5, 1998, pages 277-286, XP002940462 ISSN: 1340-2838 -& DATABASE EMBL [Online] 17 November 1998 (1998-11-17) NAGASE ET AL: "PREDICTION OF THE CODING SEQUENCES OF UNIDENTIFIED HUMAN GENES. XI. THE COMPLETE SEQUENCES OF 100 NEW CDNA CLONES FROM BRAIN WHICH CODE FOR LARGE PROTEINS IN VITRO" retrieved from EBI Database accession no. AB018314 XP002189294
- DATABASE EMBL [Online] 26 April 1999 (1999-04-26) STRAUSBERG R.: "NCI_CGAP_GC6 Homo sapiens cDNA clone IMAGE:2236894 3' similar to SW:P532_Human Q13625 p53-binding protein 53BP2" retrieved from EBI Database accession no. AI625004 XP002189295
- LOPEZ CHARLES D ET AL: "Proapoptotic p53-interacting protein 53BP2 is induced by UV irradiation but suppressed by p53." MOLECULAR AND CELLULAR BIOLOGY, vol. 20, no. 21, November 2000 (2000-11), pages 8018-8025, XP002189292 ISSN: 0270-7306
- DATABASE EMBL [Online] 27 April 1999 (1999-04-27) YANG J.P. ET AL: "IDENTIFICATION OF A NOVEL INHIBITOR OF NUCLEAR FACTOR-KAPPA B, RELA-ASSOCIATED INHIBITOR" retrieved from EBI Database accession no. AF078036 XP002202190 -& YANG J P ET AL: "IDENTIFICATION OF A NOVEL INHIBITOR OF NUCLEAR FACTOR-KAPPA B, RELA-ASSOCIATED INHIBITOR" JOURNAL OF BIOLOGICAL CHEMISTRY, AMERICAN SOCIETY OF BIOLOGICAL CHEMISTS, BALTIMORE, MD, US, vol. 274, no. 22, May 1999 (1999-05), pages 15662-15670, XP002927619 ISSN: 0021-9258
- MORI T ET AL: "Aberrant overexpression of 53BP2 mRNA in lung cancer cell lines" FEBS LETTERS, ELSEVIER SCIENCE PUBLISHERS, AMSTERDAM, NL, vol. 465, no. 2-3, 14 January 2000 (2000-01-14), pages 124-128, XP004260774 ISSN: 0014-5793
- SAMUELS-LEV YARDENA ET AL: "ASPP proteins specifically stimulate the apoptotic function of p53." MOLECULAR CELL, vol. 8, no. 4, October 2001 (2001-10), pages 781-794, XP002202189 ISSN: 1097-2765

## Description

The invention relates to members of a family of genes, which encode polypeptides capable of inhibit the pro-apoptotic activity of p53.

Tumour suppressor genes encode proteins which function to inhibit cell growth or division and are therefore important with respect to maintaining proliferation, growth and differentiation of normal cells. Mutations in tumour suppressor genes result in abnormal cell-cycle progression whereby the normal cell-cycle check points which arrest the cell-cycle, when, for example, DNA is damaged, are ignored and damaged cells divide uncontrollably. The products of tumour suppressor genes function in all parts of the cell (eg cell surface, cytoplasm, nucleus) to prevent the passage of damaged cells through the cell- cycle (ie G1, S, G2, M and cytokinesis).

A number of tumour suppressor genes have been isolated and sequenced. These include, by example only, the Retinoblastoma gene (Rb), mutations in which are linked to cancers such as bone (osteocarcoma), bladder, small cell lung and breast cancer, as well as retinoblastoma, and the Wilms Tumour - 1 gene (WT-1), mutations in which are linked to nephroblastoma and neurofibromatosis.

The tumour suppressor gene family, MAD (Mothers against *dpp* (decapentapelgic gene) and MADR (MAD related genes) have been identified in a number of species. These genes encode proteins involved in signal transduction pathways required for serine/threonine receptor signalling. MADR1 is essential for signalling of *dpp* pathway. MADR2 is another MADR and mutations in this gene have been linked with colorectal cancer (6% of sporadic colorectal cancers). The sequence of the MADR2 gene, also known as Smad2, is disclosed in WO98/07849.

Arguably the tumour suppressor gene which has been the subject of the most intense research is p53. p53 encodes a protein which functions as a transcription factor and is a key regulator of the cell division cycle. It was discovered in 1978 (Lane and Crawford, 1979) as a protein shown to bind with affinity to the SV40 large T antigen. The p53 gene encodes a 393 amino acid polypeptide with a molecular weight of 53kDa.

Genes regulated by the transcriptional activity of p53 contain a p53 recognition sequence in their 5' regions. These genes are activated when the cellular levels of p53 are elevated due to, for example DNA damage. Examples of genes which respond to p53 include, mdm2 (Momand et al 1992), Bax (Miyashita and Reed, 1995) and PIG-3 (Polyak et al, 1997). Bax and PIG-3 are involved in one of the most important functions of p53, the induction of apoptosis. Apoptosis, or programmed cell death, is a natural process which removes damaged cells. It is of importance with respect to many cellular processes, including the removal of pre-cancerous cells, cell/tissue development and homeostasis.

As mentioned above, one of the most important tumour suppression functions of p53 is its ability to induce apoptosis. The ability to up-regulate the expression of some of the pro-apoptotic genes such as Bax provided some evidence of how p53 induces apoptosis. However, by comparing the Bax expression in p53(-/-) and p53(+/+) transgenic mice and wild-type it is clear that only in a limited number of tissues was the expression of Bax regulated by p53 in response to DNA damage. Thus it remains unclear why the expression of p53 could only induce the expression of Bax in a cell type specific manner. It was shown recently that mutation in p53 can change promoter specificity. Two of the tumour-derived mutant p53 genes were shown to be defective in transactivation of the Bax promoter but competent to transactivate other promoters of p53 target genes such as mdm2 and p21wafl. These observations suggested that to be able to transactivate genes like Bax is very important for the tumour suppression function of p53.

It is known that p53 can induce apoptosis through transcriptional dependent and independent pathways. In addition, p53 induced-apoptosis can be blocked by the oncogene bcl-2. However, bcl-2 does not inhibit the transactivation function of p53. So far, very little is known about the molecular mechanisms of how bcl-2 inhibits p53- induced apoptosis.

53BP2 is a p53 binding protein initially discovered by Iwabuchi *et al* (1994). 53BP2 was isolated in a yeast 2-hybrid screen and was found to consist of 528 amino acids from the C-terminus of the protein. It contains a proline rich sequence, four ankryin repeats and an SH3 domain. Subsequently it was identified as a protein which interacted with Bcl-2 (Naumovski and Cleary, 1996). A longer version of this protein was isolated and named as bBP2/53BP2. Based on the *in vitro* translation data, the authors (Naumoviski and Cleary, 1996) predicted that the bBP2/53BP2 protein consisted of 1005 amino acids.

In an attempt to understand how the apoptotic function of p53 can be regulated *in vivo*, we generated antibodies to 53BP2 and showed that in most of the cells tested, the expression level of 53BP2 is low. We also observed that the endogenous bBP2/53BP2 unexpectedly encodes a protein larger than the 1005 amino acids predicted by Naumovski and Cleary. This protein, is called ASP-2, or ASP-2/53BP .

We have shown that the C-terminal half of bBP2/53BP2 does not have a significant effect on the activity of p53. However, ASP-2/53BP stimulated the transactivation function of p53. Most interestingly, ASP-2/53BP can specifically enhance the transactivation function of p53 on the promoters derived from pro-apoptosis related genes such as Bax and PIG-3.

Using the cDNA sequence of ASP-2, we did a BLAST search and identified a clone named as KIAA0771 with significant homology to the nucleic acid sequence encoding bBP2/53BP2 suggesting that the newly identified sequence is a member of a family of genes which encode apoptosis stimulating proteins (ASP's). This member of the family is referred to as ASP-1.

We have named the novel nucleic acid sequence ASP-1, (Apoptosis Stimulating Protein 1), which encodes a polypeptide which has sequence homology to bBP2/53BP2.

We have identified a regulator of ASP-2 which inhibits the p53-stimulatory effect of ASP-2. We have called this regulator I-ASP. I-ASP was disclosed in the art as RAI (Rel1A-associated Inhibitor) from Yang JP et al. JBC 274: 15662 (1999) and WO 0032628 as inhibitor of NF-KB. We have found that in tumours expressing ASP-1 and ASP-2, the expression of I-ASP is up-regulated compared to the matched normal controls. This suggests that the tumour suppression function of p53 may be positively and negatively regulated by ASP and I-ASP *in vivo.*

In the following, the expression "polypeptide of the invention" will refer to I-ASP.

The scope of the invention is defined exclusively by the claims, the description has illustrative purpose.

According to an aspect of the invention there is provided an antibody or binding part thereof, that binds to at least a part of the polypeptide of the invention.

In a preferred embodiment of the invention said binding part is selected from the group consisting of: F(ab')₂, Fab, Fv and Fd fragments; antibodies comprising CDR3 regions.

In a preferred embodiment of the invention said antibody is a monoclonal antibody.

In yet a further preferred embodiment of the invention said antibody is humanised.

Alternatively, said antibody is a chimeric antibody produced by recombinant methods to contain the variable region of said antibody with an invariant or constant region of a human antibody.

Chimeric antibodies are recombinant antibodies in which all of the V-regions of a mouse or rat antibody are combined with human antibody C-regions. Humanised antibodies are recombinant hybrid antibodies which fuse the complimentarity determining regions from a rodent antibody V-region with the framework regions from the human antibody V-regions. The C-regions from the human antibody are also used. The complimentarity determining regions (CDRs) are the regions within the N-terminal domain of both the heavy and light chain of the antibody to where the majority of the variation of the V-region is restricted. These regions form loops at the surface of the antibody molecule. These loops provide the binding surface between the antibody and antigen.

The-production of antibodies is well known in the art. Several laboratory text books are availalable to the skilled artisan. For example, Antibodies, Lane & Harlow , Cold Spring Harbour Laboratories.

According to a further aspect of the invention there is provided a method for determining the expression of mRNA and/or the polypeptide according to the invention.

According to a further aspect of the invention there is provided method to screen for agents capable of modulating the activity of the polypeptide according to the invention comprising:
i) providing a cell or cell-line which expresses the polypeptide according to the invention;
ii) exposing the cell to at least one agent to be tested; and
iii) monitoring the effect of the agent(s) on the activity of the polypeptide.

According to yet a further aspect of the invention there is provided a method to screen for agents capable of modulating the activity of the polypeptide according to the invention comprising:
i) providing at least the polypeptide according to the invention;
ii) exposing the polypeptide to at least one agent to be tested; and
iii) monitoring the binding of said agent(s) by said polypeptide.

In a further preferred embodiment of the invention said agent is an antagonist which inhibits the activity of the polypeptide according to the invention. Preferably said agent is a polypeptide.

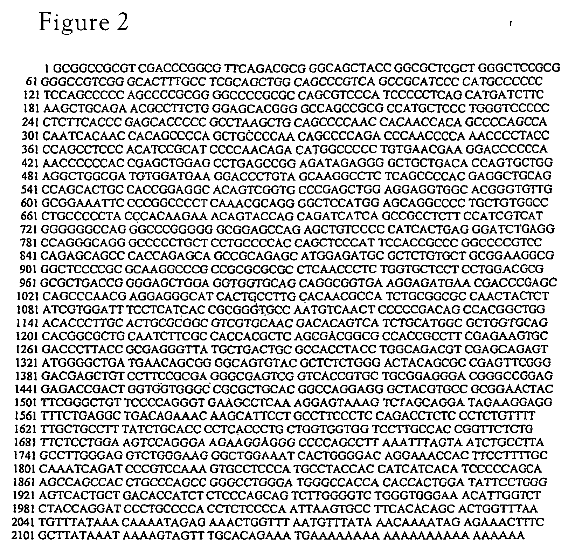

According to a further aspect of the invention there is provided an antisense nucleic acid molecule wherein said molecule comprises the antisense sequence of the sense sequence according to the invention. Preferably said antisense nucleic acid molecule comprises the antisense sequence of the sense sequence represented in Figure 2 or part thereof.

According to a further aspect of the invention there is provided a pharmaceutical composition comprising an antisense molecule according to the invention.

In a preferred embodiment of the invention said antisense nucleic acid is combined with at least one chemotherapeutic agent. Preferably said agent is an anti-cancer agent selected from the group consisting of: cisplatin; carboplatin; cyclosphosphamide; melphalan; carmusline; methotrexate; 5-fluorouracil; cytarabine; mercaptopurine; daunorubicin; doxorubicin; epirubicin; vinblastine; vincristine; dactinomycin; mitomycin C; taxol; L-asparaginase; G-CSF; an enediyne such as chalicheamicin or esperamicin; chlorambucil; ARA-C; vindesine; bleomycin; and etoposide. Other agents that can be combined with the foregoing include agents that acts on the tumor neovasculature or immunomodulators. Preferably the agent that acts on the tumor neovasculature is selected from the group consisting of combrestatin A4, angiostatin and endostatin. Preferably the immunomodulator is selected from the group consisting of α-interferon, γ-interferon, and tumor necrosis factor alpha (TNFα).

In a preferred embodiment of the invention said agent is cisplatin.

The invention thus involves dectection of I-ASP polypeptides, genes encoding I-ASP, functional modifications and variants of the foregoing, useful fragments of the foregoing, as well as therapeutics relating thereto. The expression of this gene affects apoptosis by binding to p53 and related polypeptides.

As used herein with respect to nucleic acids, the term "isolated" means: (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art.

Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art. An isolated nucleic acid as used herein is not a naturally occurring chromosome.

As used herein with respect to polypeptides, "isolated" means separated from its native environment and present in sufficient quantity to permit its identification or use. Isolated, when referring to a protein or polypeptide, means, for example: (i) selectively produced by expression cloning or (ii) purified as by chromatography or electrophoresis. Isolated proteins or polypeptides may be, but need not be, substantially pure. The term "substantially pure" means that the proteins or polypeptides are essentially free of other substances with which they may be found in nature or *in vivo* systems to an extent practical and appropriate for their intended use. Substantially pure polypeptides may be produced by techniques well known in the art. Because an isolated protein may be admixed with a pharmaceutically acceptable carrier in a pharmaceutical preparation, the protein may comprise only a small percentage by weight of the preparation. The protein is nonetheless isolated in that it has been separated from the substances with which it may be associated in living systems, i.e. isolated from other proteins.

An aspect of the invention is those nucleic acid sequences which code for I-ASP polypeptides and which hybridize to a nucleic acid molecule as provided herein, under stringent conditions. The term "stringent conditions" as used herein refers to parameters with which the art is familiar. Nucleic acid hybridization parameters may be found in references which compile such methods, e.g. *Molecular Cloning: A Laboratory Manual*, J. Sambrook, et al., eds., Second Edition, Cold Spring Harbor Laboratory Press, Cold Spring Harbor, New York, 1989, or *Current Protocols in Molecular Biology*, F.M. Ausubel, et al., eds., John Wiley & Sons, Inc., New York. More specifically, stringent conditions, as used herein, refers, for example, to hybridization at 65°C in hybridization buffer (3.5 x SSC, 0.02% Ficoll, 0.02% polyvinyl pyrrolidone, 0.02% Bovine Serum Albumin, 2.5mM NaH₂PO₄(pH7), 0.5% SDS, 2mM EDTA). SSC is 0.15M sodium chloride/0.015M sodium citrate, pH7; SDS is sodium dodecyl sulphate; and EDTA is ethylenediaminetetracetic acid. After hybridization, the membrane upon which the DNA is transferred is washed at 2 x SSC at room temperature and then at 0.1 - 0.5 X SSC/0.1 x SDS at temperatures up to 68°C.

There are other conditions, reagents, and so forth which can be used, which result in a similar degree of stringency. The skilled artisan will be familiar with such conditions, and thus they are not given here. It will be understood, however, that the skilled artisan will be able to manipulate the conditions in a manner to permit the clear identification of homologs and alleles of I-ASP nucleic acids. The skilled artisan also is familiar with the methodology for screening cells and libraries for expression of such molecules which then are routinely isolated, followed by isolation of the pertinent nucleic acid molecule and sequencing.

In general homologs and alleles typically will share at least 90% nucleotide identity and/or at least 95% amino acid identity to the disclosed nucleotide and amino acid sequences respectively, in some instances will share at least 95% nucleotide identity and/or at least 97% amino acid identity and in still other instances will share at least 98% nucleotide identity and/or at least 99% amino acid identity. The homology can be calculated using various, publicly available software tools developed by NCBI (Bethesda, Maryland) that can be obtained through the Internet (ftp:/ncbi.nlm.nih.gov/pub/).

Exemplary tools include the BLAST system available at http://www.ncbi.nlm.nih.gov, preferably using default settings. Pairwise and ClustalW alignments (BLOSUM30 matrix setting) as well as Kyle-Doolittle hydropathic analysis can be obtained using the MacVector sequence analysis software (Oxford Molecular Group). Watson-Crick complements of the foregoing nucleic acids also are embraced by the invention.

In screening for nucleic acids encoding I-ASP proteins with sequence homology to the nucleic acids described herein, a Southern blot may be performed using the foregoing conditions, together with a detectable probe (e.g., radioactive, chemiluminescent). After washing the membrane to which the DNA is finally transferred, the probe signal can be detected, such as by placing the membrane against X-ray film or phosphorimager plates to detect the radioactive signal, or by processing the membrane to detect chemiluminescent signal.

The invention also includes degenerate nucleic acids which include alternative codons to those present in the native materials. For example, serine residues are encoded by the codons TCA, AGT, TCC, TCG, TCT and AGC. Each of the six codons is equivalent for the purposes of encoding a serine residue. Thus, it will be apparent to one of ordinary skill in the art that any of the serine-encoding nucleotide triplets may be employed to direct the protein synthesis apparatus, *in vitro* or *in vivo,* to incorporate a serine residue into an elongating polypeptide. Similarly, nucleotide sequence triplets which encode other amino acid residues include, but are not limited to: CCA, CCC, CCG and CCT (proline codons); CGA, CGC, CGG, CGT, AGA and AGG (arginine codons); ACA, ACC, ACG and ACT (threonine codons); AAC and AAT (asparagine codons); and ATA, ATC and ATT (isoleucine codons). Other amino acid residues may be encoded similarly by multiple nucleotide sequences. Thus, the invention embraces degenerate nucleic acids that differ from the biologically isolated nucleic acids in codon sequence due to the degeneracy of the genetic.code.

The invention also provides modified nucleic acid molecules or polypeptides which include additions, substitutions and deletions of one or more nucleotides or amino acids. As used herein, "one or more" means 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more up to a number that does not substantially change the function of the molecule for those molecules in which the function is desired to be substantially similar to the original nucleic acid or polypeptide. A substantial change of function would be, for example, a dominant negative protein, or a protein which has lost one or more of its functions.

In preferred embodiments, these modified nucleic acid molecules and/or the polypeptides they encode retain at least one activity or function of the unmodified nucleic acid molecule and/or the polypeptides, such as p53 binding, antigenicity, transcriptional activity, etc. In certain embodiments, the modified nucleic acid molecules encode modified polypeptides, preferably polypeptides having conservative amino acid substitutions as are described elsewhere herein. The modified nucleic acid molecules are structurally related to the unmodified nucleic acid molecules and in preferred embodiments are sufficiently structurally related to the unmodified nucleic acid molecules so that the modified and unmodified nucleic acid molecules hybridize under highly stringent conditions known to one of skill in the art.

For example, modified nucleic acid molecules which encode polypeptides having single amino acid changes can be prepared. Each of these nucleic acid molecules can have one, two or three nucleotide substitutions exclusive of nucleotide changes corresponding to the degeneracy of the genetic code as described herein. Likewise, modified nucleic acid molecules which encode polypeptides having two amino acid changes can be prepared which have, e.g., 2-6 nucleotide changes. Numerous modified nucleic acid molecules like these will be readily envisioned by one of skill in the art, including for example, substitutions of nucleotides in codons encoding amino acids 2 and 3, 2 and 4, 2 and 5, 2 and 6, and so on. In the foregoing example, each combination of two amino acids is included in the set of modified nucleic acid molecules, as well as all nucleotide substitutions which code for the amino acid substitutions. Additional nucleic acid molecules that encode polypeptides having additional substitutions (i.e., 3 or more), additions or deletions (e.g., by introduction of a stop codon or a splice site(s)) also can be prepared and are embraced by the invention as readily envisioned by one of ordinary skill in the art. Any of the foregoing nucleic acids or polypeptides can be tested by routine experimentation for retention of structural relation or activity to the nucleic acids and/or polypeptides disclosed herein.

The invention also provides isolated fragments of I-ASP or complements thereof of sufficient length to represent a sequence unique within the human genome, and identifying a nucleic acid encoding I-ASP polypeptides. These fragments can be considered unique in that a unique fragment is one that is a 'signature' for the larger nucleic acid. A unique fragment, for example, is long enough to assure that its precise sequence is not found in molecules outside of I-ASP nucleic acids defined above, i.e., that it specifically identifies the I-ASP sequences. A unique fragment includes a sequence of contiguous nucleotides which is not identical to any sequence present in publicly available databases (e.g., GenBank) as of the filing date of this application, although certain fragments may contain as a portion of the fragment some previously known sequence deposited in GenBank. Likewise, complements of publicly known sequences and fragments of the publicly known sequences and complements thereof can be a portion of, but not all of the unique fragments of I-ASP. Thus a unique fragment excludes, by definition, sequences consisting solely of EST and/or gene sequences deposited in publicly available databases as of the earliest filing date of the sequences contained in this application. Thus, a unique fragment must contain a nucleotide sequence other than the exact sequence of those in GenBank or fragments thereof. The difference may be an addition, deletion or substitution with respect to the GenBank sequence or it may be a sequence wholly separate from the GenBank sequence.

Fragments of I-ASP nucleic acid molecules, including unique fragments, can be used as probes in hybridization blot assays (e.g., Southern, Northern) to identify such nucleic acids, in nuclease protection assays to measure transcription, or can be used in amplification assays such as those employing PCR. As known to those skilled in the art, large probes such as 200 250, 300 or more nucleotides are preferred for certain uses such as Southern and Northern blots, while smaller fragments will be preferred for uses such as PCR. Fragments also can be used to produce fusion proteins for generating antibodies or determining binding of the polypeptide fragments, or for generating immunoassay components. Likewise, fragments can be employed to produce nonfused fragments of the I-ASP polypeptides such as N-terminal or C-terminal fragments, or the various protein domains disclosed herein, useful, for example, in the preparation of antibodies, in immunoassays, and as a competitive binding partners of the I-ASP polypeptides and/or other polypeptides which bind to p53 or rel polypeptides, for example, in therapeutic applications. Fragments further can be used as antisense molecules, as described herein, to inhibit the expression of I-ASP nucleic acids and polypeptides, particularly for therapeutic purposes as described in greater detail herein.

As will be recognized by those skilled in the art, the size of the unique fragment will depend upon its conservancy in the genetic code. Thus, some regions of I-ASP nucleic acid molecules and their complements will require longer segments to be unique while others will require only short segments, typically between 12 and 32 nucleotides (e.g. 12, 13, 14, 15, 16, 17, 18, 19, 20, 21, 22, 23, 24, 25, 26, 27, 28, 29, 30, 31 and 32 bases long). This disclosure intends to embrace each and every fragment of each sequence, beginning at the first nucleotide, the second nucleotide and so on, up to 8 nucleotides short of the end, and ending anywhere from nucleotide number 8, 9, 10 and so on for each sequence, up to the very last nucleotide (provided the sequence is unique as described above). Many segments of the I-ASP nucleic acids, or complements thereof, that are 25 or more nucleotides in length will be unique. Those skilled in the art are well versed in methods for selecting such sequences, typically on the basis of the ability of the unique fragment to selectively distinguish the sequence of interest from non I-ASP nucleic acids. A comparison of the sequence of the fragment to those on known databases typically is all that is necessary, although *in vitro* confirmatory hybridization and sequencing analysis may be performed.

A fragment can be a functional fragment. A functional fragment of a nucleic acid molecule of the invention is a fragment which retains some functional property of the larger nucleic acid molecule, such as coding for a functional polypeptide, binding to proteins (e.g., p53), regulating transcription of operably linked nucleic acids, coding for immunologically recognized epitopes and the like. One of ordinary skill in the art can readily determine using the assays described herein and those well known in the art to determine whether a fragment is a functional fragment of a nucleic acid molecule using no more than routine experimentation.

As mentioned above, the invention embraces antisense oligonucleotides that selectively bind to a nucleic acid molecule encoding an I-ASP polypeptide, to modulate p53 binding, transcriptional activity or apoptosis, for example. This is desirable in virtually any medical condition wherein a modulation of p53 activity is desirable, such as cancer and conditions involving aberrant apoptosis.

As used herein, the term "antisense oligonucleotide" or "antisense" describes an oligonucleotide that is an oligoribonucleotide, oligodeoxyribonucleotide, modified oligoribonucleotide, or modified oligodeoxyribonucleotide which hybridizes under physiological conditions to DNA comprising a particular gene or to an mRNA transcript of that gene and, thereby, inhibits the transcription of that gene and/or the translation of that mRNA. The antisense molecules are designed so as to interfere with transcription or translation of a target gene upon hybridization with the target gene or transcript. Those skilled in the art will recognize that the exact length of the antisense oligonucleotide and its degree of complementarity with its target will depend upon the specific target selected, including the sequence of the target and the particular bases which comprise that sequence. It is preferred that the antisense oligonucleotide be constructed and arranged so as to bind selectively with the target under physiological conditions, i.e., to hybridize substantially more to the target sequence than to any other sequence in the target cell under physiological conditions. Based upon the I-ASP nucleic acid sequences provided herein, or upon allelic or homologous genomic and/or cDNA sequences, one of skill in the art can easily choose and synthesize any of a number of appropriate antisense molecules for use in accordance with the present invention. For example, a "gene walk" comprising a series of oligonucleotides of 15-30 nucleotides spanning the length of a I-ASP nucleic acid can be prepared, followed by testing for inhibition of the corresponding I-ASP expression. Optionally, gaps of 5-10 nucleotides can be left between the oligonucleotides to reduce the number of oligonucleotides synthesized and tested.

In order to be sufficiently selective and potent for inhibition, such antisense oligonucleotides should comprise at least 10 and, more preferably, at least 15 consecutive bases which are complementary to the target, although in certain cases modified oligonucleotides as short as 7 bases in length have been used successfully as antisense oligonucleotides (Wagner et al., *Nature Biotechnol.* 14:840-844, 1996). Most preferably, the antisense oligonucleotides comprise a complementary sequence of 20-30 bases. Although oligonucleotides may be chosen which are antisense to any region of the gene or mRNA transcripts, in preferred embodiments the antisense oligonucleotides correspond to N-terminal or 5' upstream sites such as translation initiation, transcription initiation or promoter sites. In addition, 3'-untranslated regions may be targeted. Targeting to mRNA splicing sites has also been used in the art but may be less preferred if alternative mRNA splicing occurs. In addition, the antisense is targeted, preferably, to sites in which mRNA secondary structure is not expected (see, e.g., Sainio et al., *Cell Mol. Neurobiol.* 14(5):439-457, 1994) and at which proteins are not expected to bind. Finally, although the I-ASP cDNA sequences are disclosed herein, one of ordinary skill in the art may easily derive the genomic DNA corresponding to these cDNAs. Thus, the present invention also provides for antisense oligonucleotides which are complementary to IASP genomic DNA. Similarly, antisense to allelic or homologous cDNAs and genomic DNAs are enabled without undue experimentation.

In one set of embodiments, the antisense oligonucleotides of the invention may be composed of "natural" deoxyribonucleotides, ribonucleotides, or any combination thereof. That is, the 5' end of one native nucleotide and the 3' end of another native nucleotide may be covalently linked, as in natural systems, via a phosphodiester internucleoside linkage. These oligonucleotides may be prepared by art recognized methods which may be carried out manually or by an automated synthesizer. They also may be produced recombinantly by vectors.

In preferred embodiments, however, the antisense oligonucleotides of the invention also may include "modified" oligonucleotides. That is, the oligonucleotides may be modified in a number of ways which do not prevent them from hybridizing to their target but which enhance their stability or targeting or which otherwise enhance their therapeutic effectiveness.

The term "modified oligonucleotide" as used herein describes an oligonucleotide in which (1) at least two of its nucleotides are covalently linked via a synthetic internucleoside linkage (i.e., a linkage other than a phosphodiester linkage between the 5' end of one nucleotide and the 3' end of another nucleotide) and/or (2) a chemical group not normally associated with nucleic acids has been covalently attached to the oligonucleotide. Preferred synthetic internucleoside linkages are phosphorothioates, alkylphosphonates, phosphorodithioates, phosphate esters, alkylphosphonothioates, phosphoramidates, carbamates, carbonates, phosphate triesters, acetamidates, carboxymethyl esters and peptides.

The term "modified oligonucleotide" also encompasses oligonucleotides with a covalently modified base and/or sugar. For example, modified oligonucleotides include oligonucleotides having backbone sugars which are covalently attached to low molecular weight organic groups other than a hydroxyl group at the 3' position and other than a phosphate group at the 5' position. Thus modified oligonucleotides may include a 2'-O-alkylated ribose group. In addition, modified oligonucleotides may include sugars such as arabinose instead of ribose. The present invention, thus, contemplates pharmaceutical preparations containing modified antisense molecules that are complementary to and hybridizable with, under physiological conditions, nucleic acids encoding I-ASP polypeptides, together with pharmaceutically acceptable carriers.

Antisense oligonucleotides may be administered as part of a pharmaceutical composition. Such a pharmaceutical composition may include the antisense oligonucleotides in combination with any standard physiologically and/or pharmaceutically acceptable carriers which are known in the art. The compositions should be sterile and contain a therapeutically effective amount of the antisense oligonucleotides in a unit of weight or volume suitable for administration to a patient. The characteristics of the carrier will depend on the route of administration. Physiologically and pharmaceutically acceptable carriers include diluents, fillers, salts, buffers, stabilizers, solubilizers, and other materials which are well known in the art.

As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to, plasmids, phagemids and virus genomes. A cloning vector is one which is able to replicate in a host cell, and which typically is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium or just a single time per host before the host reproduces by mitosis. In the case of phage, replication may occur actively during a lytic phase or passively during a lysogenic phase. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (e.g., β-galactosidase, luciferase or alkaline phosphatase), and genes which visibly affect the phenotype of transformed or transfected cells, hosts, colonies or plaques (e.g., various fluorescent proteins such as green fluorescent protein, GFP). Preferred vectors are those capable of autonomous replication and expression of the structural gene products present in the DNA segments to which they are operably joined.

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. In particular, such 5' non-transcribed regulatory sequences will include a promoter region which includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art.

Expression vectors containing all the necessary elements for expression are commercially available and known to those skilled in the art. See, e.g., Sambrook et al.*, Molecular Cloning: A Laboratory Manual,* Second Edition, Cold Spring Harbor Laboratory Press, 1989. Cells are genetically engineered by the introduction into the cells of heterologous DNA (RNA) encoding a I-ASP polypeptide or fragment or variant thereof. That heterologous DNA (RNA) is placed under operable control of transcriptional elements to permit the expression of the heterologous DNA in the host cell.

Preferred systems for mRNA expression in mammalian cells are those such as pcDNA3.1 and pRc/CMV (available from Invitrogen, Carlsbad, CA) that contain a selectable marker such as a gene that confers G418 resistance (which facilitates the selection of stably transfected cell lines) and the human cytomegalovirus (CMV) enhancer-promoter sequences. Additionally, suitable for expression in primate or canine cell lines is the pCEP4 vector (Invitrogen), which contains an Epstein Barr virus (EBV) origin of replication, facilitating the maintenance of plasmid as a multicopy extrachromosomal element. Another expression vector is the pEF-BOS plasmid containing the promoter of polypeptide Elongation Factor 1α, which stimulates efficiently transcription *in vitro*. The plasmid is described by Mishizuma and Nagata (*Nuc. Acids Res.* 18:5322, 1990), and its use in transfection experiments is disclosed by, for example, Demoulin (*Mol. Cell. Biol*. 16:4710-4716, 1996). Still another preferred expression vector is an adenovirus, described by Stratford-Perricaudet, which is defective for E1 and E3 proteins (*J. Clin. Invest.* 90:626-630, 1992). The use of the adenovirus as an Adeno.P1A recombinant is disclosed by Warnier et al., in intradermal injection in mice for immunization against P1A (*Int. J. Cancer,* 67:303-310, 1996).

The isolation of the I-ASP nucleic acid molecules also makes it possible for the artisan to diagnose a disorder characterized by expression (or relative lack thereof) of these molecules. These methods involve determining expression of the I-ASP nuclei acids, and/or polypeptides derived therefrom. In the former situation, such determinations can be carried out via any standard nucleic acid determination assay, including the polymerase chain reaction as exemplified in the examples below, or assaying with labeled hybridization probes.

The invention also involves agents such as polypeptides which bind to I-ASP polypeptides and to complexes of I-ASP polypeptides and binding partners such as p53. Such binding agents can be used, for example, in screening assays to detect the presence or absence of I-ASP polypeptides and complexes of I-ASP polypeptides and their binding partners and in purification protocols to isolate I-ASP polypeptides and complexes of I-ASP polypeptides and their binding partners. Such agents also can be used to inhibit the native activity of the I-ASP polypeptides or their binding partners, for example, by binding to such polypeptides, or their binding partners or both.

The invention, therefore, embraces peptide binding agents which, for example, can be antibodies or fragments of antibodies having the ability to selectively bind to I-ASP polypeptides. Antibodies include polyclonal and monoclonal antibodies, prepared according to conventional methodology.

Significantly, as is well-known in the art, only a small portion of an antibody molecule, the paratope, is involved in the binding of the antibody to its epitope (see, in general, Glark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. Similarly, an antibody from which the Fc region has been enzymatically cleaved, or which has been produced without the Fc region, designated a Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation. Within the antigen-binding portion of an antibody, as is well-known in the art, there are complementarity determining regions (CDRs), which-directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity.

It is now well-established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. See, e.g., U.S. patents 4,816,567, 5,225,539, 5,585,089, 5,693,762 and 5,859,205.

Thus, for example, PCT International Publication Number WO 92/04381 teaches the production and use of humanized murine RSV antibodies in which at least a portion of the murine FR regions have been replaced by FR regions of human origin. Such antibodies, including fragments of intact antibodies with antigen-binding ability, are often referred to as "chimeric" antibodies.

Fully human monoclonal antibodies also can be prepared, for example, by immunization of non-human animals transgenic for human immunoglobulin genes. See, for example, U.S. patents 5,814,318, 5,877,397, 6,091,001, 6,114,598.

Thus, as will be apparent to one of ordinary skill in the art, the present invention also provides for F(ab')₂, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')₂ fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

Thus, the invention involves polypeptides of numerous size and type that bind specifically to I-ASP polypeptides, and complexes of both I-ASP polypeptides and their binding partners. These polypeptides may be derived also from sources other than antibody technology. For example, such polypeptide binding agents can be provided by degenerate peptide libraries which can be readily prepared in solution, in immobilized form or as phage display libraries. Combinatorial libraries also can be synthesized of peptides containing one or more amino acids: Libraries further can be synthesized of peptoids and non-peptide synthetic moieties.

Phage display can be particularly effective in identifying binding peptides useful according to the invention. Briefly, one prepares a phage library (using e.g. m13, fd, or lambda phage), displaying inserts from 4 to about 80 amino acid residues using conventional procedures. The inserts may represent, for example, a completely degenerate or biased array. One then can select phage-bearing inserts which bind to the I-ASP polypeptide. This process can be repeated through several cycles of reselection of phage that bind to the I-ASP polypeptide. Repeated rounds lead to enrichment of phage bearing particular sequences. DNA sequence analysis can be conducted to identify the sequences of the expressed polypeptides. The minimal linear portion of the sequence that binds to the I-ASP polypeptide can be determined. One can repeat the procedure using a biased library containing inserts containing part or all of the minimal linear portion plus one or more additional degenerate residues upstream or downstream thereof Yeast two-hybrid screening methods also may be used to identify polypeptides that bind to the or I-ASP polypeptides. Thus, the I-ASP polypetides of the invention, or fragments thereof, can be used to screen peptide libraries, including phage display libraries, to identify and select peptide binding partners of the I-ASP polypeptides of the invention. Such molecules can be used, as described, for screening assays, for purification protocols, for interfering directly with the functioning of I-ASP and for other purposes that will be apparent to those of ordinary skill in the art.

The invention further provides efficient methods of identifying pharmacological agents or lead compounds for agents active at the level of a I-ASP modulatable cellular function. In particular, such functions include p53 binding, and apoptosis. Generally, the screening methods involve assaying for compounds which interfere with I-ASP activity such as p53 binding, etc, although compounds which enhance I-ASP activity also can be assayed using the screening methods. Such methods are adaptable to automated, high throughput screening of compounds. The target therapeutic indications for pharmacological agents detected by the screening methods are limited only in that the target cellular function be subject to modulation by alteration of the formation of a complex comprising a I-ASP polypeptide or fragment thereof and one or more natural I-ASP intracellular binding targets, such as p53. Target indications include apoptosis.

A wide variety of assays for pharmacological agents are provided; including, labeled *in vitro* protein-protein binding assays, electrophoretic mobility shift assays, immunoassays, cell-based assays such as two- or three-hybrid screens, expression assays; etc. For example, hybrid screens are used to rapidly examine the effect of transfected nucleic acids on the intracellular binding of I-ASP polypeptides or fragments thereof to specific intracellular targets. The transfected nucleic acids can encode, for example, combinatorial peptide libraries or antisense molecules. Convenient reagents for such assays, e.g., GAL4 fusion proteins, are known in the art. An exemplary cell-based assay involves transfecting a cell with a nucleic acid encoding a I-ASP polypeptide fused to a GAL4 DNA binding domain and a nucleic acid encoding a p53 domain which interacts with I-ASP fused to a transcription activation domain such as VP16. The cell also contains a reporter gene operably linked to a gene expression regulatory region, such as one or more GAL4 binding sites. Activation of reporter gene transcription occurs when the ASP and p53 fusion polypeptides bind such that the GAL4 DNA binding domain and the VP16 transcriptional activation domain are brought into proximity to enable transcription of the reporter gene. Agents which modulate a I-ASP polypeptide mediated cell function are then detected through a change in the expression of reporter gene. Methods for determining changes in the expression of a reporter gene are known in the art.

I-ASP fragments used in the methods, when not produced by a transfected nucleic acid are added to an assay mixture as an isolated polypeptide. I-ASP polypeptides preferably are produced recombinantly, although such polypeptides may be isolated from biological extracts. Recombinantly produced I-ASP polypeptides include chimeric proteins comprising a fusion of a I-ASP protein with another polypeptide, e.g., a polypeptide capable of providing or enhancing protein-protein binding, sequence specific nucleic acid binding (such as GAL4), enhancing stability of the I-ASP polypeptide under assay conditions, or providing a detectable moiety, such as green fluorescent protein or Flag epitope.

The assay mixture is comprised of a natural intracellular I-ASP binding target such as p53 or a fragment thereof capable of interacting with I-ASP. While natural I-ASP binding targets may be used, it is frequently preferred to use portions (e.g., peptides or nucleic acid fragments) or analogs (i.e., agents which mimic the I-ASP binding properties of the natural binding target for purposes of the assay) of the I-ASP binding target so long as the portion or analog provides binding affinity and avidity to the I-ASP fragment measurable in the assay.

The assay mixture also comprises a candidate pharmacological agent. Typically, a plurality of assay mixtures are run in parallel with different agent concentrations to obtain a different response to the various concentrations. Typically, one of these concentrations serves as a negative control, i.e., at zero concentration of agent or at a concentration of agent below the limits of assay detection. Candidate agents encompass numerous chemical classes, although typically they are organic compounds. Preferably, the candidate pharmacological agents are small organic compounds, i.e., those having a molecular weight of more than 50 yet less than about 2500, preferably less than about 1000 and, more preferably, less than about 500. Candidate agents comprise functional chemical groups necessary for structural interactions with polypeptides and/or nucleic acids, and typically include at least an amine, carbonyl, hydroxyl or carboxyl group, preferably at least two of the functional chemical groups and more preferably at least three of the functional chemical groups. The candidate agents can comprise cyclic carbon or heterocyclic structure and/or aromatic or polyaromatic structures substituted with one or more of the above-identified functional groups. Candidate agents also can be biomolecules such as peptides, saccharides, fatty acids, sterols, isoprenoids, purines, pyrimidines, derivatives or structural analogs of the above, or combinations thereof and the like. Where the agent is a nucleic acid, the agent typically is a DNA or RNA molecule, although modified nucleic acids as defined herein are also contemplated.

Candidate agents are obtained from a wide variety of sources including libraries of synthetic or natural compounds. For example, numerous means are available for random and directed synthesis of a wide variety of organic compounds and biomolecules, including expression of randomized oligonucleotides, synthetic organic combinatorial libraries, phage display libraries of random peptides, and the like. Alternatively, libraries of natural compounds in the form of bacterial, fungal, plant and animal extracts are available or readily produced. Additionally, natural and synthetically produced libraries and compounds can be readily be modified through conventional chemical, physical, and biochemical means. Further, known pharmacological agents may be subjected to directed or random chemical modifications such as acylation, alkylation, esterification, amidification, etc. to produce structural analogs of the agents.

A variety of other reagents also can be included in the mixture. These include reagents such as salts, buffers, neutral proteins (e.g., albumin), detergents, etc. which may be used to facilitate optimal protein-protein and/or protein-nucleic acid binding. Such a reagent may also reduce non-specific or background interactions of the reaction components. Other reagents that improve the efficiency of the assay such as protease, inhibitors, nuclease inhibitors, antimicrobial agents, and the like may also be used.

The mixture of the foregoing assay materials is incubated under conditions whereby, but for the presence of the candidate pharmacological agent, the I-ASP polypeptide specifically binds the cellular binding target, a portion thereof or analog thereof. The order of addition of components, incubation temperature, time of incubation, and other perimeters of the assay may be readily determined. Such experimentation merely involves optimization of the assay parameters, not the fundamental composition of the assay. Incubation temperatures typically are between 4°C and 40°C. Incubation times preferably are minimized to facilitate rapid; high throughput screening, and typically are between 0.1 and 10 hours.

After incubation, the presence or absence of specific binding between the I-ASP polypeptide and one or more binding targets is detected by any convenient method available to the user. For cell free binding type assays, a separation step is often used to separate bound from unbound components. The separation step may be accomplished in a variety of ways. Conveniently, at least one of the components is immobilized on a solid substrate, from which the unbound components may be easily separated. The solid substrate can be made of a wide variety of materials and in a wide variety of shapes, e.g., microtiter plate, microbead, dipstick, resin particle, etc. The substrate preferably is chosen to maximum signal to noise ratios, primarily to minimize background binding, as well as for ease of separation and cost.

Separation may be effected for example, by removing a bead or dipstick from a reservoir, emptying or diluting a reservoir such as a microtiter plate well, rinsing a bead, particle, chromatographic column or filter with a wash solution or solvent The separation step preferably includes multiple rinses or washes. For example, when the solid substrate is a microtiter plate, the wells may be washed several times with a washing solution, which typically includes those components of the incubation mixture that do not participate in specific bindings such as salts, buffer, detergent, non-specific protein, etc. Where the solid substrate is a magnetic bead, the beads may be washed one or more times with a washing solution and isolated using a magnet.

Detection may be effected in any convenient way for cell-based assays such as two-or three-hybrid screens. The transcript resulting from a reporter gene transcription assay of I-ASP polypeptide interacting with a target molecule typically encodes a directly or indirectly detectable product, e.g., β-galactosidase activity, luciferase activity, and the like. For cell free binding assays, one of the components usually comprises, or is coupled to, a detectable label. A wide variety of labels can be used, such as those that provide direct detection (e.g., radioactivity, luminescence, optical or electron density, etc). or indirect detection (e.g., epitope tag such as the FLAG epitope, enzyme tag such as horseradish peroxidase, etc.). The label may be bound to a I-ASP binding partner, or incorporated into the structure of the binding partner.

A variety of methods may be used to detect the label, depending on the nature of the label and other assay components. For example, the label may be detected while bound to the solid substrate or subsequent to separation from the solid substrate. Labels may be directly detected through optical or electron density, radioactive emissions, nonradiative energy transfers, etc. or indirectly detected with antibody conjugates, strepavidin-biotin conjugates, etc. Methods for detecting the labels are well known in the art.

The invention provides I-ASP-specific binding agents, methods of identifying and making such agents, and their use in diagnosis, therapy and pharmaceutical development. For example, I-ASP-specific pharmacological agents are useful in a variety of diagnostic and therapeutic applications, especially where disease or disease prognosis is associated with improper utilization of a pathway involving I-ASP, e.g., apoptosis, etc. Novel, I-ASP-specific binding agents include I-ASP-specific antibodies and other natural intracellular binding agents identified with assays such as two hybrid screens, and non-natural intracellular binding agents identified in screens of chemical libraries and the like.

In general, the specificity of I-ASP binding to a binding agent is shown by binding equilibrium constants. Targets which are capable of selectively binding an I-ASP polypeptide preferably have binding equilibrium constants of at least about 10⁷ M⁻¹, more preferably at least about 10⁸ M⁻¹, and most preferably at least about 10⁹ M⁻¹. The wide variety of cell based and cell free assays may be used to demonstrate I-ASP-specific binding. Cell based assays include one, two and three hybrid screens, assays in which I-ASP-mediated transcription is inhibited or increased, etc. Cell free assays include I-ASP-protein binding assays, immunoassays, etc. Other assays useful for screening agents which bind I-ASP polypeptides include fluorescence resonance energy transfer (FRET), and electrophoretic mobility shift analysis (EMSA).

Various techniques may be employed for introducing nucleic acids of the invention into cells, depending on whether the nucleic acids are introduced *in vitro* or *in vivo* in a host. Such techniques include transfection of nucleic acid-CaPO₄ precipitates, transfection of nucleic acids associated with DEAE, transfection with a retrovirus including the nucleic acid of interest, liposome mediated transfection, and the like. For certain uses, it is preferred to target the nucleic acid to particular cells. In such instances, a vehicle used for delivering a nucleic acid of the invention into a cell (e.g., a retrovirus, or other virus; a liposome) can have a targeting molecule attached thereto. For example, a molecule such as an antibody specific for a surface membrane protein on the target cell or a ligand for a receptor on the target cell can be bound to or incorporated within the nucleic acid delivery vehicle. For example, where liposomes are employed to deliver the nucleic acids of the invention, proteins which bind to a surface membrane protein associated with endocytosis may be incorporated into the liposome formulation for targeting and/or to facilitate uptake. Such proteins include capsid proteins or fragments thereof tropic for a particular cell type, antibodies for proteins which undergo internalization in cycling, proteins that target intracellular localization and enhance intracellular half life, and the like. Polymeric delivery systems also have been used successfully to deliver nucleic acids into cells, as is known by those skilled in the art. Such systems even permit oral delivery of nucleic acids.

When administered, the therapeutic compositions of the present invention are administered in pharmaceutically acceptable preparations. Such preparations may routinely contain pharmaceutically acceptable concentrations of salt, buffering agents, preservatives, compatible carriers, supplementary immune potentiating agents such as adjuvants and cytokines and optionally other therapeutic agents, such as chemotherapeutic agents.

The therapeutics of the invention can be administered by any conventional route, including injection or by gradual infusion over time. The administration may, for example, be oral, intravenous, intraperitoneal, intramuscular, intracavity, subcutaneous, or transdermal. When antibodies are used therapeutically, a preferred route of administration is by pulmonary aerosol. Techniques for preparing aerosol delivery systems containing antibodies are well known to those of skill in the art. Generally, such systems should utilize components which will not significantly impair the biological properties of the antibodies, such as the paratope binding capacity (see, for example, Sciarra and Cutie, "Aerosols," in Remington's Pharmaceutical Sciences, 18th edition, 1990, pp 1694-1712; incorporated by reference). Those of skill in the art can readily determine the various parameters and conditions for producing antibody aerosols without resort to undue experimentation. When using antisense preparations of the invention, slow intravenous administration is preferred.

The compositions of the invention are administered in effective amounts. An "effective amount" is that amount of a composition that alone, or together with further doses, produces the desired response. In the case of treating a particular disease, such as cancer, the desired response is inhibiting the progression of the disease. This may involve only slowing the progression of the disease temporarily, although more preferably, it involves halting the progression of the disease permanently. This can be monitored by routine methods or can be monitored according to diagnostic methods of the invention discussed herein.

Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons; psychological reasons or for virtually any other reasons.

The pharmaceutical compositions used in the foregoing methods preferably are sterile and contain an effective amount of antibody for producing the desired response in a unit of weight or volume suitable for administration to a patient. The response can, for example, be measured by determining the signal transduction enhanced or inhibited by the composition via a reporter system as described herein, by measuring downstream effects such as gene expression, or by measuring the physiological effects of the composition, such as regression of a tumor, decrease of disease symptoms, modulation of apoptosis, etc. Likewise, the effects of antisense I-ASP molecules can be readily determined by measuring expression of the individual genes in cells to which an antisense composition is added. Other assays will be known to one of ordinary skill in the art and can be employed for measuring the level of the response.

The doses of antibody administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

In general, doses of antibody are formulated and administered in doses between 1 ng and 1 mg, and preferably between 10 ng and 100 µg, according to any standard procedure in the art. Other protocols for the administration of antibody compositions will be known to one of ordinary skill in the art, in which the dose amount, schedule of injections, sites of injections, mode of administration (e.g., intra-tumoral) and the like vary from the foregoing. Administration of antibody compositions to mammals other than humans, e.g. for testing purposes or veterinary therapeutic purposes, is carried out under substantially the same conditions as described above. A subject, as used herein, is a mammal, preferably a human, and including a non-human primate, cow, horse, pig, sheep, goat, dog, cat or rodent.

When administered, the pharmaceutical preparations of the invention are applied in pharmaceutically-acceptable amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

Antibody compositions may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application. The components of the pharmaceutical compositions also are capable of being co-mingled with the molecules of the present invention, and with each other, in a manner such that there is no interaction which would substantially impair the desired pharmaceutical efficacy.

The pharmaceutical compositions may contain suitable buffering agents, including: acetic acid in a salt; citric acid in a salt; boric acid in a salt; and phosphoric acid in a salt.

The pharmaceutical compositions also may contain, optionally, suitable preservatives, such as: benzalkonium chloride; chlorobutanol; parabens and thimerosal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions suitable for parenteral administration conveniently comprise a sterile aqueous or non-aqueous preparation of antibodies, which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

In another aspect of the invention, antibodies are used in the manufacture of a medicament for modulating apoptosis. The medicament can be placed in a vial and be incorporated into a kit to be used for treating a subject. In certain embodiments, other medicaments which modulate the same responses or which favorably affect the antibody compositions can also be included in the same kit, such as chemotherapeutic agents. The kits can include instructions or other printed material on how to administer the antibody compositions and any other components of the kit.

An embodiment of the invention will now be described, by example only, and with reference to the following examples and figures and table;

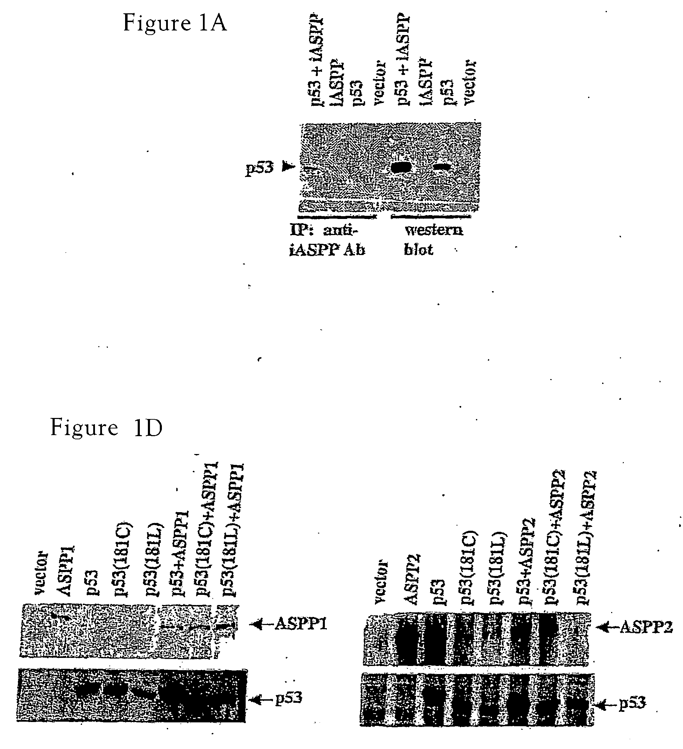

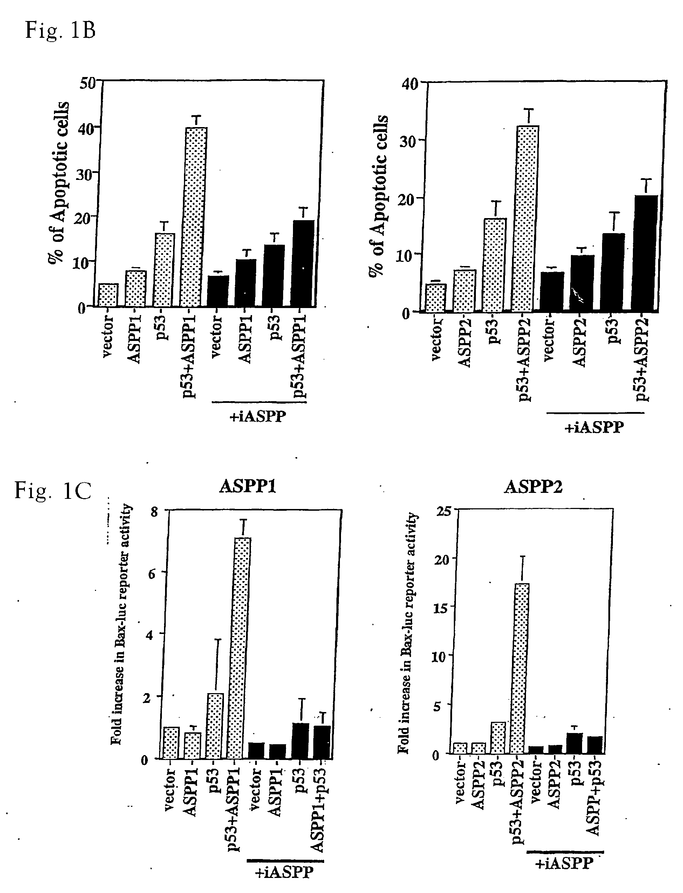

Figure 1 Saos2 cells were transfected with either vector, p53 (5µg), I-ASP (10µg) or p53+ I-ASP and then incubated for 16hrs. The cells were lysed in NP40 lysis buffer and 1000 µg of lysate subject to an immunoprecipitation performed with polyclonal antibodies to I-ASP bound to Protein G beads. The presence of p53 was detected by western blotting of the immunocomplexes using rabbit polyclonal p53 antibody CM1, Figure 1A. Saos2 cells were transfected with either ASP-1 (8µg) or ASP-2 (4µg), I-ASP (5µg) and p53 (50ng). 40 µl of the corresponding lysates were run on a 10% gel, ASP-1 was detected with V5 antibody, ASP-2 with DX.5410, I-ASP with mouse anti I-ASP antibody, p53 with DO1 and PCNA with anti-PCNA antibody, Figure 1D.; Figure 1B shows the induction of p53 induced apoptosis by ASP-1 and ASP-2 and the inhibition of p53-induced apoptosis by I-ASP; Figure 1C shows the activation of p53 responsive promoter, Bax by ASP-1 and ASP-2 and inhibition of transactivation by I-ASP.
Figure 2 represents the DNA sequence of I-ASP;

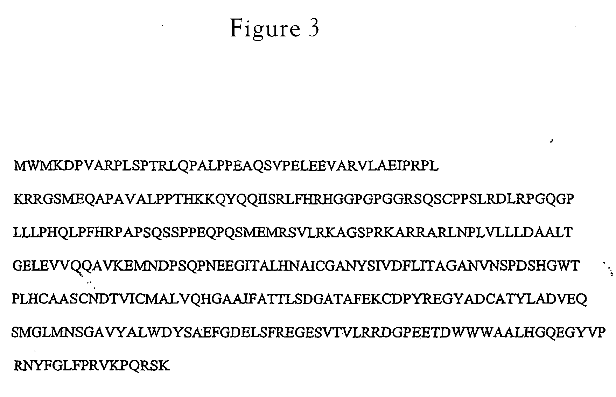

Figure 3 represents the protein sequence of I-ASP;

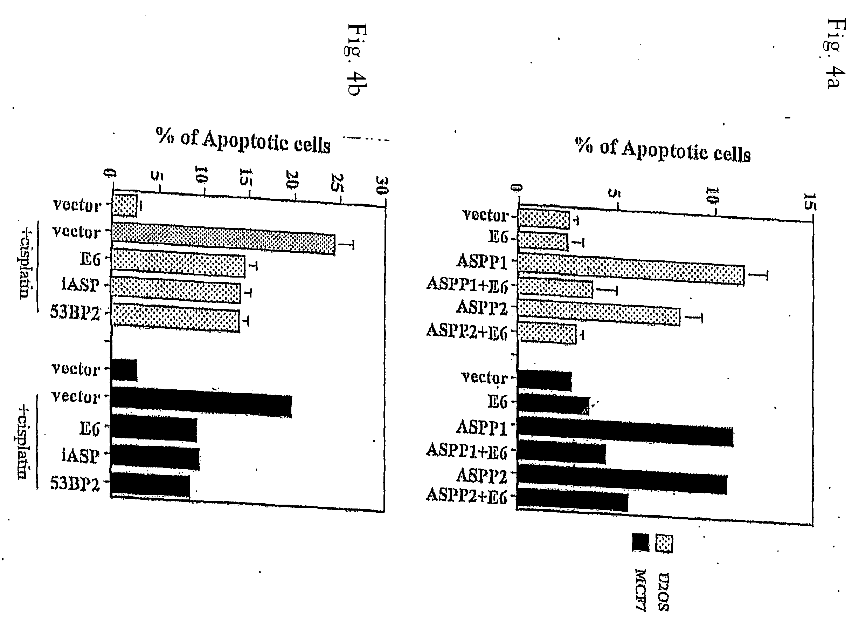

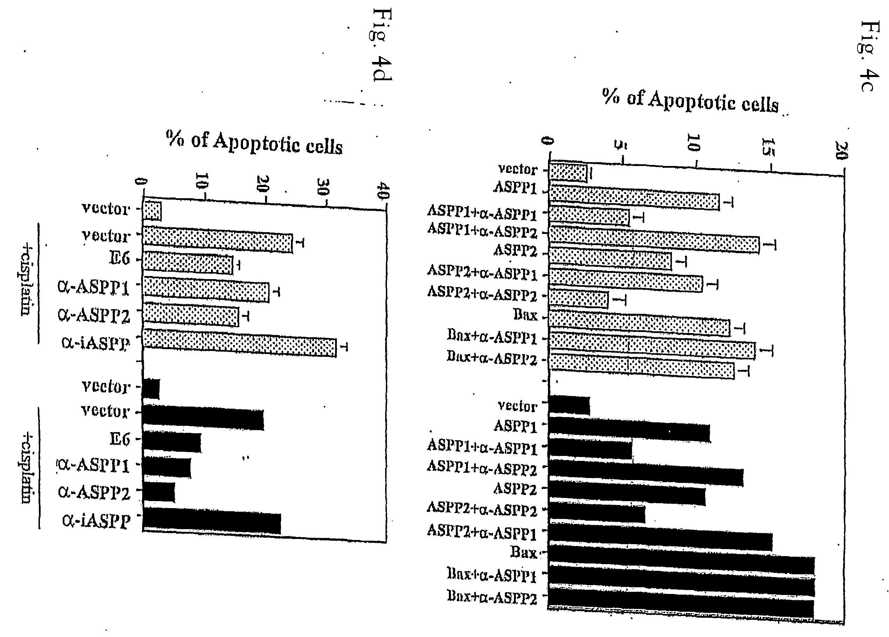

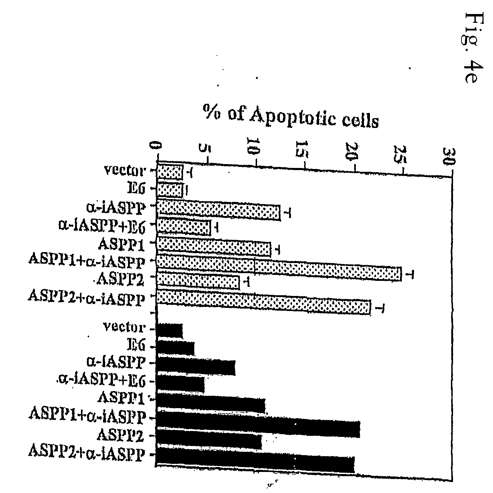

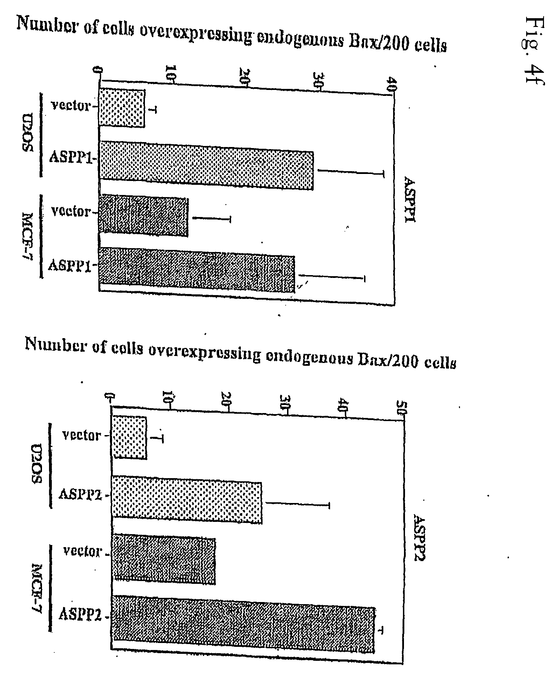

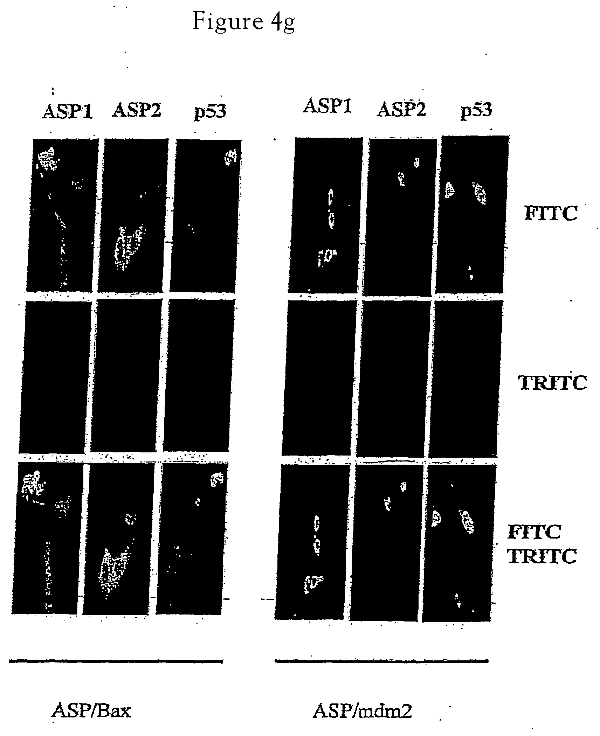

Figure 4 shows that the apoptotic function of p53 is highly regulated by ASP family members *in vivo.* Wild type p53 expressing cell lines U2OS and MCF-7 were transfected with plasmids expressing proteins as indicated together with a cell surface marker CD20 (A-E). The transfected cells were gated and analysed by FACS. The bar graphs represent the percentage of transfected cells with sub-G1 DNA content, characteristic of apoptosis. The plasmids expressing antisense RNA of ASP-1, ASP-2 and I-ASP are labelled as α-ASP-1, α-ASP-2 and α-I-ASP respectively. The viral oncoprotein E6 of human HPV16 is indicated as E6. In figures 4B and 4D, the cells were transfected with the plasmids as indicated. Subsequently, the transfected U2OS and MCF-7 cells were incubated with medium containing cisplatin at concentrations of 5 and 3µg/ml respectively. 30 hours later, cells were harvested and analysed as above. For F and G, both U2OS and MCF-7 cells were transfected with plasmids expressing proteins as indicated. The co-expression of ASP or p53 and endogenous Bax or mdm2 were visualised after cell fixation, by double immunofluoresence labelling as indicated in figure 4G. For figure 4F, 200 U2OS or MCF-7 cells transfected either with vector alone or ASPP plasmids were examined for the overexpression of endogenous Bax protein. The bar graphs represent the mean value of at least three independent experiments;
Figure 5A illustrates a model describing the interaction of ASP family members with p65 , IkB and p53; figure 5C and 5D is a graphical representation of the ability of IkB affectr the transactivation function of p53 on Bax and mdm2 promoters in the presence and absence of ASP-2;
Figure 6A is a graphical representation of the ability of wild-type p65 and deletion mutant Δp65 to transactivate a NFkB reporter plasmid; Figure 6B is a graphical representation of the induction of apoptosis in cells expressing and co-expressing p53, ASP-2, p65 and Δp65
Figure 7A illustrates the enhancing effect of I-ASP on the transfoming function of E7;
Figure 7B illustrates the enhancing effect of I-ASP on cell resistance to cisplatin; and

Table 1 represents a summary of mRNA expression of ASP-1, ASP-2 and I-ASP in 40 pairs of normal and tumour matched RNA samples derived from Grade I and II breast tumours expressing wild-type p53.

### Example 1

A recently isolated sequence, reL Associated Inhibitor (RAI) was identified as a p65 rel A binding protein which contains 315 amino acids. RAI does not have the α-helical domain of ASP-1 or ASP-2 but it does contain the proline rich region, the anlayin repeats and the SH3 domain. The p53 contact residues of ASP-2 are also conserved in RAI. To investigate the activity of RAI which we have named I-ASP, we cloned the coding sequence into a mammalian expression vector pcDNA3. We also synthesised a peptide (RLQPALPPEAQSVPELEE) found in I-ASP which does not have sequence similarity to ASP-1 and ASP-2. A mouse antibody specific to this unique I-ASP peptide did not cross react with either ASP-1 or ASP-2 (data not shown). Using this mouse anti-I-ASP specific antibody to immunoprecipitate the transfected I-ASP in Saos-2 cells, we were able to demonstrate that I-ASP can also interact with p53 (figure 1A). Like the ASP-2 mutant, 53BP2/ASP-2 (600-1128), the expression of I-ASP did not induce apoptosis on its own. When I-ASP was co-expressed with p53, it had a small inhibitory effect on the apoptotic function of p53. The most significant effect of I-ASP on the apoptotic function of p53 was observed when ASP-1 or ASP-2 were co-expressed. In agreement with our prediction, the co-expression of I-ASP inhibited the enhanced apoptotic function of p53 effected by ASP-1 and ASP-2 (figure 1B). Similarly, co-expression of I-ASP together with ASP-1 or ASP-2 was able to completely abolish the ability of both ASP-1 and ASP-2 to stimulate the transactivation function of p53 on the Bax promoter (figure 1C). The co-expression of I-ASP did not significantly alter the expression levels of either p53 or ASP (figure 1D). These results indicated that *in vivo* the pro-apoptotic function of ASP-1 and ASP-2 may be regulated by the natural inhibitor I-ASP. Thus the balance between the expression levels of ASP-1, ASP-2 and I-ASP may determine cell fate, ie whether a cell lives or dies.

### Example 8

### Example 2

In contrast to ASP-1 and ASP-2, the expression level of I-ASP was generally low in the normal and human breast tumour tissue samples tested. However, overexpression of I-ASP was detected in 8 of the tumour tissues compared to their normal paired controls (Table 1). The most striking phenomenon was the correlation of the normal expression of ASP-1 and ASP-2 with the overexpression of I-ASP. 7 of the I-ASP overexpressing tumours did not have any down regulation of ASP-1 and ASP-2 expression (Table 1). This result supports the arguement for the biological importance of I-ASP as the natural inhibitor of ASP-1 and ASP-2 *in vivo*.

### Example 3

To study the roles that endogenous ASP family members play in regulating apoptosis induced by endogenous p53, we introduced ASP-1 or ASP-2 into the cell lines U2OS and MCF7 which express wild-type p53. When expressed in these cells ASP-1 and ASP-2 induced apoptosis (figure 4A). The viral oncoprotein E6, which is derived from human papilloma virus and which can bind and specifically target p53 for degradation, inhibited the apoptosis induced by ASP-1 or ASP-2, demonstrating that ASP-1 and ASP-2 can induce p53-dependent apoptosis.

We also studied the dominant negative function of 53BP2 and I-ASP in inhibiting apoptosis induced by endogenous p53 in response to DNA damage. Before exposure to cisplatin, U2OS and MCF7 cells were transfected with plasmids encoding HPV16 E6, I-ASP, or 53BP2. The percentage of transfected cells dying of apoptosis in response to the treatment with cisplatin was then determined by FACS analysis (figure 4B). Treatment with cisplatin induced over 20% of the transfected cells to die of apoptosis. The expression of E6 reduced the percentage of apoptotic cells to below 15% suggesting that cisplatin induces p53-dependent apoptosis in U2OS cells. In agreement with this, expression of I-ASP or 53BP2 was able to inhibit cisplatin-induced apoptosis to a similar extent as E6. These results suggest that the apoptotic function of endogenous p53 can be regulated by the expression of ASP family members.

To demonstrate further that endogenous ASP family members do play significant roles in regulating the apoptotic function of p53, we used an antisense approach. We cloned fragments from the 5'ends of the ASP-1, ASP-2 and 1-ASP cDNAs into a mammalian expression vector in an antisense orientation and tested the ability of the antisense RNA to specifically inhibit the protein synthesis of ASP family members *in vitro* (data not shown). Expression of antisense ASP-1 only inhibited apoptosis induced by ASP-1 but not by ASP-2. Similarly, expression of antisense ASP-2 only inhibited apoptosis induced by ASP-2 but not ASP-1. The specific effect of antisense ASP-1 and ASP-2 was further supported by the observation that co-expression of antisense ASP-1 or ASP-2 did not influence apoptosis mediated by Bax under the same conditions (figure 4C). Hence it allowed us to investigate the role of endogenous ASP-1 and ASP-2 in regulating the apoptotic function of endogenous p53 in response to DNA damage. U2OS and MCF-7 cells were transfected with the various expression plasmids prior to the treatment with cisplatin. FACS analysis showed that around 20-30% of control transfected cells undergo apoptosis. Expression of E6 reduced the percentage of apoptotic cells to half, indicating that cisplatin can induce apoptosis through both p53 dependent and independent pathways in these cells. Expression of antisense RNA of ASP-1 or ASP-2 inhibited cisplatin-induced apoptosis to the same extent as E6 (figure 4D), similar to the effects seen with 53BP2 and I-ASP. This suggests that endogenous ASP-1 and ASP-2 play important roles in regulating the apoptotic function of p53 in response to DNA damage.

We suspect that the stimulatory effect of the endogenous ASP-1 and ASP-2 on p53 induced apoptosis in response to cisplatin may be under-estimated due to high levels of I-ASP detected in these cells (data not shown) which could prevent ASP-1 and ASP-2 from enhancing the apoptotic function of p53. To study the anti-apoptotic role of I-ASP, both U2OS and MCF-7 cells were transfected with antisense I-ASP. Antisense I-ASP induced p53-dependent apoptosis that was abrogated by the co-expression of E6. Removal of the anti-apoptotic function of I-ASP by antisense I-ASP also enhanced the apoptotic function of ASP-1 and ASP-2 (figure 4E). Unlike antisense ASP-1 and ASP-2, the expression of antisense I-ASP did not inhibit cisplatin-induced apoptosis. A small increase in apoptotic cells was consistently detected (figure 4D). Those results demonstrated that ASP-1 and ASP-2 specifically stimulate the apoptotic function of p53 *in vivo.* Endogenous I-ASP functions as an inhibitor of ASP and can block apoptosis induced by endogenous p53.

The antisense nucleic acid molecules were derived from the cDNAs of ASP-1, ASP-2 and I-ASP and were amplified by PCR on the respective plasmid clones using primers spanning the following nucleotide regions (relative to the initial ATG): -74 to 923; -253 to 839 and -37 to 536 for ASP-1, ASP-2 and I-ASP respectively. The amplified segments were purified with the QIAquick PCR purification kit (QIAGEN) and ligated in the pcDNA3.1/V5-His TOPO vector (Invitrogen) according to the manufacturer's instructions. The plasmids containing antisense DNA of ASP-1, ASP-2 and I-ASP were produced by means of the QIAGEN plasmid purification kit according to manufacturers instructions.

### Example 4

It has been well documented that p53 and p65RelA of NF kappaB play very important roles in regulating apoptosis in response to stress. However, very little is known about how these two apoptotic pathways can work together *in vivo*. Attempts had been made towards the understanding of whether there is a cross-talk between p53 and NF-kappaB. The first evidence came from the recent observation that p53 can induce the DNA binding activity of p65 Rel A. Most importantly Ikb, the inhibitor of p65 Rel A, can inhibit the apoptotic function of p53. However it was not clear how p53 can induce the DNA binding activity of p65 and how Ikb can inhibit the apoptotic function of p53.

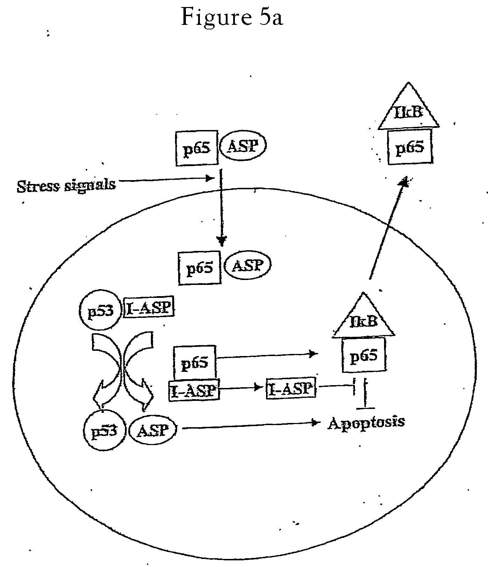

Interestingly, it was discovered very recently that both ASP-2 and I-ASP can interact with p65 rel A, a component of NF-kappaB, in a yeast hybrid assay. I-ASP can also inhibit the transactivation function of p65, although less effectively than Ikb. The region required for ASP-2 and I-ASP to interact with rel A p65 is very similar as that required for p53. Therefore it is possible that there might be some competition between p53 and p65 rel A to interact with ASP-2 and I-ASP. Since ASP family members happen to be the common partner between p53 and p65, we speculated that ASP family members may connect the apoptotic function of p53 and NF-kappaB. In this working model (figure 5A), p53 may induce the DNA binding activity of p65 by interacting with the nuclear I-ASP and allow p65 to bind DNA. In addition, Ikb could inhibit p53-induced apoptosis by binding to p65 and releasing I-ASP. The increased nuclear concentration of I-ASP can then interact with p53 and prevent ASP-2 or ASP-1 to stimulate the transactivation function of p53. If this hypothesis is correct, one would expect that the expression of Ikb should have a profound effect on p53 - induced apoptosis in the presence of ASP-1 or ASP-2.

The results shown in figure 5B are consistent with this notion. In the Ikb-expressing cells, 7.2% of the cells die of apoptosis compared to 4.6% of cells transfected with in vector alone transfected cells. The effect of Ikb on p53-induced apoptosis was also minimal since the percentage of apoptotic cells detected in p53 versus p53+Ikb expressing cells were 12% and 11 % respectively. This could be due to the very low level of ASP-1 and ASP--2 expression in Saos-2 cells. In agreement with the results shown before, the co-expression of ASP-2 produced a significant enhancement of p53 induced apoptosis. The percentage of apoptotic cells in p53+ASP2 transfected cells was 30%. Interestingly, it is under this setting, the co-expression of Ikb showed the most profound effect. The co-expression of Ikb was able to reduce the amount of apoptotic cells induced by p53 and ASP-2 from 30% to 16%. This result suggested that Ikb could inhibit p53-induced apoptosis by preventing ASP-2 to stimulate p53 function. Similar results were also obtained when Ikb was co-expressed with p53 and ASP-1.

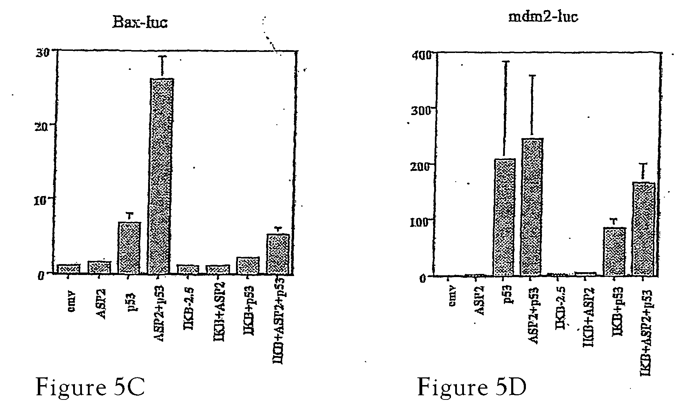

We have shown that ASP-2 can enhance the apoptotic function of p53 by specifically stimulating the transactivation function of p53 on the promoters of pro-apoptotic genes such as Bax. Thus we also investigated the effect of Ikb on the transactivation function of p53 on the Bax and mdm2 promoters in the presence or absence of ASP2, see Figures 5C and D. As shown previously, co-expression of ASP-2 and p53 stimulated the transactivation function of p53 by about 8-fold. Under the same conditions, the expression of Ikb did not show any detectable inhibition on the Bax promoter reporter activity suggesting that Ikb does not inhibit the transcriptional activity of Bax promoter non-specifically in Saos-2 cells. The co-expression of 50ng of Ikb with p53 only showed a very little inhibition on the transactivation function of p53. However, when Ikb, ASP-2 and p53 were co-expressed, Ikb was able to inhibit the ASP-2 mediated stimulation of p53 transactivation function dramatically (figure 5D).

We have shown that ASP-1 and ASP-2 can specifically stimulate the transactivation function of p53 on the Bax promoter but not the mdm2 promoter. Hence, if Ikb was indeed inhibiting the transactivation function of p53 via ASP-2 specifically, it would not be able to show a significant inhibition on the promoter activity of mdm2. Under the same conditions, we also tested the ability of Ikb to inhibit the transactivation function of p53 on the mdm2 promoter activity. As shown in figure 5D, the co-expression of ASP-2 had very little effect on the transactivation function of p53 on the mdm2 promoter. Most importantly, Ikb hardly inhibited the transactivation function of p53 on the mdm2 promoter even in the presence of ASP-2. The results shown here suggest for the first time that Ikb may inhibit the apoptotic function of p53 by preventing ASP-1 or ASP-2 from stimulating the transactivation function of p53.

To further investigate the role of the ASP family in connecting with the p53 and the NFkb pathway, we also studied the effect of the ASP-2 and p65 relA interaction on the apoptotic function of p53. Based on the working model in figure 5A, the p65/ASP interaction may facilitate the nuclear entry of ASP protein, thus allowing the p53/ASP interaction and the release of nuclear I-ASP to bind to the nuclear p65. The residues 176-406 of p65 binds to ASP-2 and I-ASP.

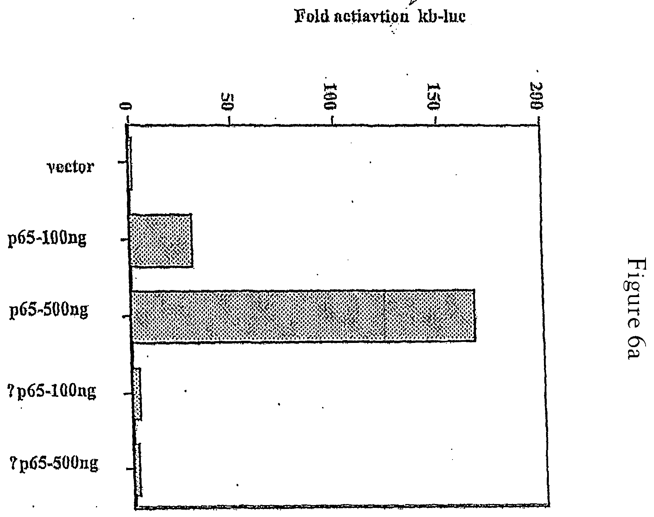

As a transcription factor, p65 can transactivate many target genes. Since p53-induced apoptosis requires p65 and is also correlated with the increased DNA-binding activity of NFkB, it suggested that the DNA-binding activity of p65 may be essential to co-operate with p53 to induce apoptosis. Being able to bind both p53 and p65 places the ASP family of proteins in a central role. One possibility is that ASP binding to p65 may be the mediator for the p53 induced DNA binding activity of p65. However, the co-expression of p53 failed to induce the transcriptional activity of p65 on its reporter. The co-expression of p53 and ASP-2 also failed to show any significant effect on the transactivation function of p65. This result suggested that ASP was not the messenger which delivers the signals from p53 to p65. Nevertheless, ASP could enable p65 to co-operate with p53 to induce apoptosis. We tested whether the action of ASP needed the DNA-binding activity of p65 NFkB. One hundred amino acids of p65 were removed from the N-terminus which contains the DNA binding region of p65. The Δp65 construct thus generated was transcriptionally inactive when tested on the NFkb reporter plasmid. If the ASP-p65 interaction is the mediator of p53 and NFkB pathways, Δp65 would have similar effect on the apoptotic function of p53 as the full-length p65. Otherwise, Δp65 would be inactive since it is defective to transactivate any of its target genes (figure 6A). The result shown in figure 6B showed that not only both p65 and Δp65 stimulated the apoptotic function of p53 in the presence of ASP2, but that Δp65 was even more active than p65 in enhancing the apoptotic function of p53. This may due to the fact that Δp65 is more nuclear than p65. The data obtained from Δp65 argue strongly that p65 can influence the apoptotic function of p53 independent of the DNA-binding activity of p65. Hence, the interaction of ASP-2-p65 could be the mechanism of action.

### Example 5

### Example 6

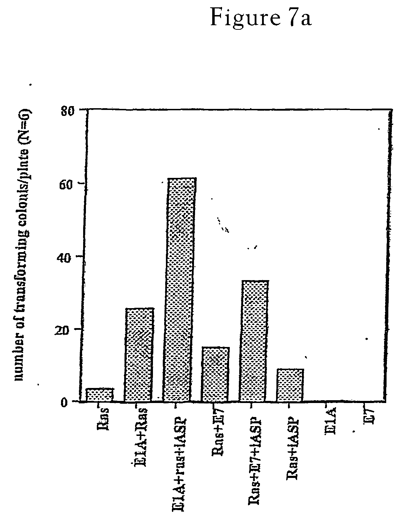

So far we have shown that I-ASP can inhibit p53-induced apoptosis in various cell lines and that its expression level is up-regulated in breast carcinoma cells *in vivo*. All these data suggest that I-ASP could be an oncogene. Since the tumour suppression function of p53 is best linked to its ability to induce apoptosis, it is likely that inhibition of p53-induced apoptosis can remove the tumour suppression function of p53. To test the oncogenic function of I-ASP, rat embryo fibroblasts were transfected with plasmids expressing I-ASP and the oncoprotein, E7. The expression of I-ASP enhanced the transforming function of E7 significantly (figure 7A). This demonstrated that I-ASP is indeed an oncogene.

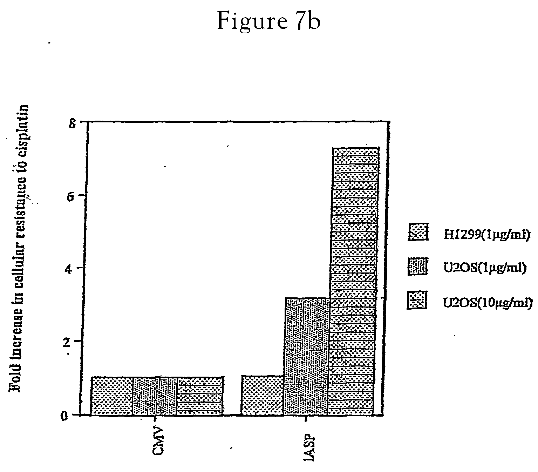

As most of the chemotherapy drugs are DNA-damage agents and induce apoptosis via p53-dependent pathways, we reasoned that the ability of I-ASP to inhibit p53-induced apoptosis may make cells more resistant to the cytotoxic effect of chemotherapy drugs such as cisplatin. MCF-7 cells (a human breast cancer cell line) were transfected with an I-ASP-expressing plasmid. The cellular resistance to the cytotoxic effect of cisplatin were compared between I-ASP-expressing and non-expressing I-ASP MCF-7 cells. The results in figure 7B shows that the expression of I-ASP can enhance the cellular resistance by about 2.5 fold. Such an increase in cellular resistance to cisplatin is biologically very significant with respect to cancer treatment. Hence, the high level of expression of I-ASP not only explains why wild type p53 is not functional in some of the human tumour cells. It may also predict the tumour response to treatments. Finally, using I-ASP overexpressing cells may allow the future identification of a more effective chemotherapy drugs.

### References

Iwabuchi, K., Li, B., Bartel, P., and Fields, S. (1993). Use of the two-hybrid system to identify the domain of p53 involved in oligomerization. Oncogene 8, 1693-1696. Lane, D. P., and Crawford, L. V. (1979). T-antigen is bound to host protein in SV40-transformed cells. Nature 278, 261-263.
Ludwig, R. T., Bates, S., and Vousden, K. H. (1996). Differential activation of target cellular promoters by p53 mutants with impaired apoptotic function. Mol. Cell. Biol. 16, 4952-4960.
Miyashita, T., and Reed, J. C. (1995). Tumour suppressor p53 is a direct transcriptional activator of the human bax gene. Cell 80, p293-299.
Momand, J., Zambetti, G. P., Oslon, D. C., George, D., and Levine, A. J. (1992). The mdm-2 oncogene product forms a complex with p53 protein and inhibits p53-mediated transactivation. Cell 69, 1237-1245.
Naumovski, L., and Cleary, M. L. (1996). The p53-binding protein 53BP2 also interacts with bcl2 and impedes cell cycle progression at G2/M. Mol. Cell. Biol. 16, 3884-3892.
Polyak, K., Xia, Y., Zweier, J. L., Kinzler, K. W., and Vogelstein, B. (1997). A model for p53-induced apoptosis. Nature 389, 300-305.

**Table 1. mRNA expression of ASPP in wild type p53 expressing human breast tumour samples (grade I and II)**

| Tumour | ASP1 | ASP2 | I-ASP |
|---|---|---|---|
| 1 | ↓ | + | - |
| 2 | ↓ | + | - |
| 3 | ↓ | + | - |
| 4 | ↓ | + | - |
| 5 | ↓ | + | - |
| 6 | ↓ | + | - |
| 7 | ↓ | ↓ | - |
| 8 | + | + | ↑ |
| 9 | ↓ | ↓ | - |
| 10 | ↓ | ↓ | - |
| 11 | ↓ | + | - |
| 12 | ↓ | ↓ | - |
| 13 | ↓ | ↓ | - |
| 14 | ↓ | + | - |
| 15 | ↓ | ↓ | - |
| 16 | ↓ | ↓ | - |
| 17 | + | + | ↑ |
| 18 | ↓ | + | - |
| 19 | ↓ | + | - |
| 20 | + | + | ↑ |
| 21 | + | + | - |
| 22 | + | + | - |
| 23 | ↓ | + | - |
| 24 | + | + | - |
| 25 | + | + | ↑ |
| 26 | + | ↓ | - |
| 27 | ↓ | ↓ | - |
| 28 | + | + | ↑ |
| 29 | + | + | - |
| 30 | ↓ | + | - |
| 31 | + | + | - |
| 32 | + | + | ↑ |
| 33 | ↓ | + | - |
| 34 | ↓ | + | - |
| 35 | + | + | - |
| 36 | + | + | - |
| 37 | + | + | - |
| 38 | + | + | ↑ |
| 39 | ↓ | + | - |
| 40 | ↓ | + | ↑ |

### SEQUENCE LISTING

<110> Ludwig Institute for Cancer Research
<120> Suppressor Genes
<130> P38080WO
<140> PCT/GB01/03524
   <141> 2001-08-06
<150> 0019018.1
   <151> 2000-08-04
<160> 7
<170> PatentIn version 3.1
<210> 1
   <211> 4834
   <212> DNA
   <213> Homo sapiens
<400> 1
<210> 2
   <211> 4402
   <212> DNA
   <213> Homo sapiens
<400> 2
<210> 3
   <211> 2156
   <212> DNA
   <213> Homo sapiens
<400> 3
<210> 4
   <211> 350
   <212> PRT
   <213> Homo sapiens
<400> 4
   Met Trp Met Lys Asp Pro Val Ala Arg Pro Leu Ser Pro Thr Arg Leu
   1 5 10 15
   Gln Pro Ala Leu Pro Pro Glu Ala Gln Ser Val Pro Glu Leu Glu Glu
   20 25 30
   Val Ala Arg Val Leu Ala Glu Ile Pro Arg Pro Leu Lys Arg Arg Gly
   35 40 45
   Ser Met Glu Gln Ala Pro Ala Val Ala Leu Pro Pro Thr His Lys Lys
   50 55 60
   Gln Tyr Gln Gln Ile Ile Ser Arg Leu Phe His Arg His Gly Gly Pro
   65 70 75 80
   Gly Pro Gly Gly Arg Ser Gln Ser Cys Pro Pro Ser Leu Arg Asp Leu
   85 90 95
   Arg Pro Gly Gln Gly Pro Leu Leu Leu Pro His Gln Leu Pro Phe His
   100 105 110
   Arg Pro Ala Pro Ser Gln Ser Ser Pro Pro Glu Gln Pro Gln Ser Met
   115 120 125
   Glu Met Arg Ser Val Leu Arg Lys Ala Gly Ser Pro Arg Lys Ala Arg
   130 135 140
   Arg Ala Arg Leu Asn Pro Leu Val Leu Leu Leu Asp Ala Ala Leu Thr
   145 150 155 160
   Gly Glu Leu Glu Val Val Gln Gln Ala Val Lys Glu Met Asn Asp Pro
   165 170 175
   Ser Gln Pro Asn Glu Glu Gly Ile Thr Ala Leu His Asn Ala Ile Cys
   180 185 190
   Gly Ala Asn Tyr Ser Ile Val Asp Phe Leu Ile Thr Ala Gly Ala Asn
   195 200 205
   Val Asn Ser Pro Asp Ser His Gly Trp Thr Pro Leu His Cys Ala Ala
   210 215 220
   Ser Cys Asn Asp Thr Val Ile Cys Met Ala Leu Val Gln His Gly Ala
   225 230 235 240
   Ala Ile Phe Ala Thr Thr Leu Ser Asp Gly Ala Thr Ala Phe Glu Lys
   245 250 255
   Cys Asp Pro Tyr Arg Glu Gly Tyr Ala Asp Cys Ala Thr Tyr Leu Ala
   260 265 270
   Asp Val Glu Gln Ser Met Gly Leu Met Asn Ser Gly Ala Val Tyr Ala
   275 280 285
   Leu Trp Asp Tyr Ser Ala Glu Phe Gly Asp Glu Leu Ser Phe Arg Glu
   290 295 300
   Gly Glu Ser Val Thr Val Leu Arg Arg Asp Gly Pro Glu Glu Thr Asp
   305 310 315 320
   Trp Trp Trp Ala Ala Leu His Gly Gln Glu Gly Tyr Val Pro Arg Asn
   325 330 335
   Tyr Phe Gly Leu Phe Pro Arg Val Lys Pro Gln Arg Ser Lys
   340 345 350
<210> 5
   <211> 18
   <212> PRT
   <213> Homo sapiens
<400> 5
   Arg Leu Gln Pro Ala Leu Pro Pro Glu Ala Gln Ser Val Pro Glu Leu
   1 5 10 15
   Glu Glu
<210> 6
   <211> 1593
   <212> PRT
   <213> human
<400> 6
<210> 7
   <211> 1446
   <212> PRT
   <213> human
<400> 7

## Claims

1. An assay to diagnose cancer **characterized by** up regulation of a polypeptide in an isolated tissue sample, which polypeptide is an inhibitor of p53 apoptotic activity, wherein said polypeptide is encoded by a nucleic acid molecule selected from the group consisting of:
i) a DNA molecule consisting of a sequence as represented by Figure 2;
ii) a DNA molecule that hybridizes under stringent hybridization conditions to the nucleic acid sequence in Figure 2 and which encodes an inhibitor of p53 apoptotic activity; and
iii) a DNA molecule which is degenerate as a result of the genetic code to DNA sequences defined in (i) and (ii);
wherein said assay comprises the steps of:
a) providing an isolated tissue sample to be tested;
b) providing conditions suitable for the detection of the polypeptide by an agent and detecting the presence of the polypeptide by the agent; and
c) comparing the up-regulation of the polypeptide to matched normal controls.

2. An assay according to claim 1 wherein the tissue sample is breast tissue.

3. An assay according to claim 1 or 2 wherein the agent is an antibody.

4. An assay according to claim 3 wherein the assay is an immunoassay.

5. An assay to diagnose cancer **characterized by** up-regulation of a nucleic acid molecule in an isolated breast tissue sample, which nucleic acid encodes an inhibitor of p53 apoptotic activity, wherein said molecule is selected from the group consisting of:
i) a nucleic acid molecule consisting of a sequence as represented by Figure 2;
ii) a nucleic acid molecule that hybridizes to the nucleic acid sequence in Figure 2 and which encodes an inhibitor of p53 apoptotic activity; and
iii) a nucleic acid molecule which is degenerate as a result of the genetic code to the sequences defined in (i) and (ii);
wherein said assay comprises the steps of:
a) providing sample to be tested;
b) providing conditions suitable for the detection of said nucleic acid molecule by said agent and detecting the presence of the nucleic acid molecule by the agent; and
c) comparing the up-regulation of the nucleic acid to matched normal controls.

6. An assay according to claim 5 wherein the agent is a nucleic acid molecule capable of hybridizing to said nucleic acid sequence.

7. An assay according to claim 6 wherein the nucleic acid is at least one oligonucleotide.

8. An assay according to claim 6 or 7 wherein the assay is a polymerase chain reaction assay.

9. Use of an antibody capable of binding to a polypeptide as represented by the amino acid sequence in Figure 3, for the manufacture of a medicament for use in the treatment of cancer by the induction of apoptosis.

10. Use of an antibody according to claim 9 wherein the cancer is breast cancer.

11. Use of an antisense nucleic acid molecule comprising an antisense sequence of a sense sequence selected from the group consisting of:
i) a nucleic acid molecule consisting of a sequence as represented in Figure 2;
ii) a nucleic acid molecule consisting of a sequence which hybridizes to the nucleic acid sequence in Figure 2; and
iii) a nucleic acid molecule which consists of a sequence which is degenerate as a result of the genetic code to the nucleic acid sequences defined in (i) and (ii), for use in the manufacture of a medicament for the treatment of cancer by the induction of apoptosis.

12. A screening method for the identification of pharmacological agents that interfere with the binding of a polypeptide with p53 comprising:
i) providing a polypeptide encoded by a nucleic acid molecule selected from the group consisting of:
a) a DNA molecule consisting of a sequence as represented by the nucleic acid sequence in Figure 2;
b) a DNA molecule which hybridizes to the nucleic acid sequence in Figure 2 and which encodes an inhibitor of p53 apoptotic activity; and
c) a DNA molecule which is degenerate as a result of the genetic code to DNA sequences defined in (i) and (ii);
ii) providing at least one candidate agent;
iii) providing a preparation forming a combination of (i) and (ii);
iv) detecting or measuring the activity of the agent with respect to interfering with the binding of said polypeptide to p53; and optionally
v) testing the effect of the agent on apoptosis of a selected cell type.

13. A method according to claim 12 wherein said polypeptide is encoded by the nucleic acid sequence represented in Figure 2.

14. A method according to claim 12 or 13 wherein said polypeptide is expressed by a cell.

15. A method according to claim 14 wherein said cell is a cancer cell.

16. A method according to any of claims 12-15 wherein said polypeptide is over-expressed.

17. A method according any of claims 14-16 wherein said cell is transformed/transfected with a nucleic acid molecule encoding said polypeptide.

18. A combined pharmaceutical preparation comprising at least one antisense nucleic acid molecule of the sense sequence selected from the group consisting of:
i) a nucleic acid molecule consisting of sequence as represented in Figure 2;
ii) a nucleic acid molecule consisting of a sequence which hybridizes to the sequence in Figure 2; and
iii) a nucleic acid molecule which consists of a sequence which is degenerate as a result of the genetic code to the DNA sequences defined in (i) and (ii) and a chemotherapeutic agent.

19. A preparation according to Claim 18 wherein said chemotherapeutic agent is an anti-cancer agent selected from the group consisting of: cisplatin; carboplatin; cyclosphosphamide; melphalan; carmusline; methotrexate; 5-fluorouracil; cytarabine; mercaptopurine; daunorubicin; doxorubicin; epirubicin; vinblastine; vincristine; dactinomycin; mitomycin C; taxol; L-asparaginase; G-CSF; an enediyne such as chalicheamicin or esperamicin; chlorambucil; ARA-C; vindesine; bleomycin; or etoposide.

20. A preparation according to Claim 19 wherein said agent is cisplatin.

21. A combined pharmaceutical preparation comprising an antibody capable of binding a polypeptide encoded by a nucleic acid molecule selected from the group consisting of:
i) a nucleic acid molecule consisting of sequence as represented in Figure 2;
ii) a nucleic acid molecule consisting of a sequence which hybridizes to a nucleic acid sequence in Figure 2; and
iii) a nucleic acid molecule which consists of a sequence which is degenerate as a result of the genetic code to the DNA sequences defined in (i) and (ii) and a chemotherapeutic agent.

22. A preparation according to Claim 21 wherein said chemotherapeutic agent is selected from the group consisting of: cisplatin; carboplatin; cyclosphosphamide; melphalan; carmusline; methotrexate; 5-fluorouracil; cytarabine; mercaptopurine; daunorubicin; doxorubicin; epirubicin; vinblastine; vincristine; dactinomycin; mitomycin C; taxol; L-asparaginase; G-CSF; an enediyne such as chalicheamicin or esperamicin; chlorambucil; ARA-C; vindesine; bleomycin; or etoposide.

23. A preparation according to Claim 22 wherein said chemotherapeutic agent is cisplatin.

## Patentansprüche

1. Test zur Diagnose von Krebs, **gekennzeichnet durch** Hochregulation eines Polypeptids in einer isolierten Gewebeprobe, wobei das Polypeptid ein Inhibitor der apoptotischen Aktivität von p53 ist und wobei das Polypeptid von einem Nukleinsäuremolekül codiert wird, das ausgewählt ist aus der Gruppe bestehend aus:
i) einem DNA-Molekül, das aus einer wie in Figur 2 dargestellten Sequenz besteht;
ii) einem DNA-Molekül, das unter stringenten Hybridisierungsbedingungen an die Nukleinsäuresequenz in Figur 2 hybridisiert und das für einen Inhibitor der apoptotischen Aktivität von p53 codiert; und
iii) einem DNA-Molekül, das aufgrund des genetischen Codes gegenüber den in (i) und (ii) definierten DNA-Sequenzen degeneriert ist;
wobei der Test die Schritte umfasst:
a) Bereitstellen einer zu testenden, isolierten Gewebeprobe;
b) Bereitstellen von Bedingungen, die zum Nachweis des Polypeptids mit einem Mittel geeignet sind, und Nachweisen des Vorhandenseins des Polypeptids mit dem Mittel; und
c) Vergleichen der Hochregulation des Polypeptids mit paraltelisierten, normalen Kontrollen.

2. Test nach Anspruch 1, wobei die Gewebeprobe Srustgewehe ist.

3. Test nach Anspruch 1 oder 2, wobei das Mittel ein Antikörper ist.

4. Test nach Anspruch 3, wobei der Test ein Immunoassay ist.

5. Test zur Diagnose von Krebs, **gekennzeichnet durch** Hochregulation eines Nukleinsäuremoleküls in einer isolierten Brustgewebeprobe, wobei die Nukleinsäure für einen Inhibitor der apoptotischen Aktivität von p53 codiert und wobei das Molekül ausgewählt ist aus der Gruppe bestehend aus:
i) einem Nukleinsäuremolekül, das aus einer wie in Figur 2 dargestellten Sequenz besteht;
ii) einem Nukleinsäuremolekül, das an die Nukleinsäuresequenz in Figur 2 hybridisiert und das für einen Inhibitor der apoptotischen Aktivität von p53 codiert; und
üi) einem Nukleinsäuremolekül, das aufgrund des genetischen Codes gegenüber den in (i) und (ii) definierten Sequenzen degeneriert ist;
wobei der Test die Schritte umfasst:
a) Bereitstellen einer zu testenden Probe;
b) Bereitstellen von Bedingungen, die zum Nachweis des Nukleinsäuremoleküls mit einem Mittel geeignet sind, und Nachweisen des Vorhandenseins des Nukleinsäuremoleküls mit dem Mittel; und
c) Vergleichen der Hochregulation der Nukleinsäure mit parallelisierten, normalen Kontrollen.

6. Test nach Anspruch 5, wobei das Mittel ein Nukleinsäuremolekül ist, das an die Nukleinsäuresequenz hybridisieren kann.

7. Test nach Anspruch 6, wobei die Nukleinsäure wenigstens ein Oligonukleotid ist.

8. Test nach Anspruch 6 oder 7, wobei der Test ein Test mittels Polymerasekettenreaktion ist.

9. Verwendung eines Antikörpers, der an ein Polypeptid, wie es durch die Aminosäuresequenz in Figur 3 dargestellt ist, binden kann, zur Herstellung eines Medikaments zur Verwendung bei der Behandlung von Krebs durch die Induktion von Apoptose.

10. Verwendung eines Antikörpers nach Anspruch 9, wobei der Krebs Brustkrebs ist.

11. Verwendung eines *Antisense*-Nukleinsäuremoleküls, umfassend eine *Antisense-*Sequenz zu einer *Sense*-Sequenz, die ausgewählt ist aus der Gruppe bestehend aus:
i) einem Nukleinsäuremolekül, das aus einer wie in Figur 2 dargestellten Sequenz be steht;
ii) einem Nukleinsäuremolekül, das aus einer Sequenz besteht, die an die Nukleinsäuresequenz in Figur 2 hybridisiert;
iii) einem Nukleinsäuremolekül, das aus einer Sequenz besteht, die aufgrund des genetischen Codes gegenüber den in (i) und (ii) definierten Nukleinsäuresequenzen degeneriert ist;
zur Herstellung eines Medikaments zur Behandlung von Krebs durch die Induktion von Apoptose.

12. Screening-Verfahren zur Identifizierung von pharmakologischen Mitteln, welche die Bindung eines Polypeptids mit p53 stören, umfassend:
i) Bereitstellen eines Polypeptids, das von einem Nukleinsäuremolekül codiert wird, das ausgewählt ist aus der Gruppe bestehend aus:
a) einem DNA-Molekül, das aus einer Sequenz besteht, wie sie durch die Nukleinsäuresequenz in Figur 2 dargestellt ist;
b) einem DNA-Molekül, das an die Nukleinsäuresequenz in Figur 2 hybridisiert und das für einen Inhibitor der apoptotischen Aktivität von p53 codiert; und
c) einem DNA-Molekül, das aufgrund des genetischen Codes gegenüber den in (i) und (ii) definierten DNA-Sequenzen degeneriert ist;
ii) Bereitstellen wenigstens eines zu prüfenden Mittels;
iii) Bereitstel en einer eine Kombination aus (i) und (ii) bildenden Zubereitung;
iv) Nachweisen oder Messen der Aktivität des Mittels bezüglich seines störenden Einflusses auf die Bindung des Polypeptids an p53; und gegebenenfalls
v) Untersuchen der Wirkung des Mittels auf die Apoptose eines ausgewählten Zelltyps.

13. Verfahren nach Anspruch 12, wobei das Polypeptid von der in Figur 2 dargestellten Nuldeinsäuresequenz codiert wird.

14. Verfahren nach Anspruch 12 oder 13, wobei das Polypeptid von einer Zelle exprimiert wird.

15. Verfahren nach Anspruch 14, wobei die Zelle eine Krebszelle ist.

16. Verfahren nach irgendeinem der Ansprüche 12-15, wobei das Polypeptid überexprimiert wird.

17. Verfahren nach irgendeinem der Ansprüche 14-16, wobei die Zelle mit einem Nukleinsäuremolekül transformiert/transfiziert ist, das für dieses Polypeptid codiert.

18. Pharmazeutisches Kombinationspräparat, umfassend wenigstens ein *Antisense-*Nukleinsäuremolekül zu der *Sense*-Sequenz, die ausgewählt ist aus der Gruppe bestehend aus:
i) einem Nukleinsäuremolekül, das aus einer wie in Figur 2 dargestellten Sequenz besteht;
ii) einem Nukleinsäuremolekül, das aus einer Sequenz besteht, die an die Sequenz in Figur 2 hybridisiert; und
iii) einem Nukleinsäuremolekül, das aus einer Sequenz besteht, die aufgrund des genetischen Codes gegenüber den in (i) und (ii) definierten DNA-Sequenzen degeneriert ist;
und ein chemotherapeutisches Mittel.

19. Präparat nach Anspruch 18, wobei das chemotherapeutische Mittel ein Antikrebsmittel ist, das ausgewählt ist aus der Gruppe bestehend aus: Cisplatin; Carboplatin; Cyclosphosphamid; Melphalan; Carmustin; Methotrexat; 5-Fluoruracil; Cytarabin; Mercaptopurin; Daunorubicin; Doxorubicin; Epirubicin; Vinblastin; Vincristin; Dactinomycin; Mitomycin C; Taxol; L-Asparaginase; G-CSF; einem Endiin wie Calicheamicin oder Esperamicin; Chlorambucil; ARA-C; Vindesin; Bleomycin; oder Etoposid.

20. Präparat nach Anspruch 19, wobei das Mittel Cisplatin ist.

21. Pharmazeutisches Kombintaionspräparat, umfassend einen Antikörper, der ein Polypeptid binden kann, das von einem Nukleinsäuremolekül codiert wird, welches ausgewählt ist aus der Gruppe bestehend aus:
i) einem Nukleinsäuremolekül, das aus einer wie in Figur 2 dargestellten Sequenz besteht;
ii) einem Nukleinsäuremolekül, das aus einer Sequenz besteht, die an eine Nukleinsäuresequenz in Figur 2 hybridisiert; und
iii) einem Nukleinsäuremolekül, das aus einer Sequenz besteht, die aufgrund des genetischen Codes gegenüber den in (i) und (ii) definierten Sequenzen degeneriert ist;
und ein chemotherapeutisches Mittel.

22. Präparat nach Anspruch 21, wobei das chemotherapeutische Mittel ausgewählt ist aus der Gruppe bestehend aus: Cisplatin; Carboplatin; Cyclosphosphamid; Melphalan; Carmustin; Methotrexat; 5-Fluoruracil; Cytarabin; Mercaptopurin; Daunorubicin; Doxorubicin; Epirubicin; Vinblastin; Vincristin; Dactinomycin; Mitomycin C; Taxol; L-Asparaginase; G-CSF; einem Endiin wie Calicheamicin oder Esperamicin; Chlorambucil; ARA-C; Vindesin; Bleomycin; oder Etoposid.

23. Präparat nach Anspruch 22, wobei das chemotherapeutische Mittel Cisplatin ist.

## Revendications

1. Etude pour le diagnostic de cancer, **caractérisée en ce qu'**elle inclut une régulation d'un Polypeptide dans un échantillon de tissu isolé, lequel polypeptide est un inhibiteur de l'activité apoptotique p53, tandis que ledit polypeptide est encodé par une molécule acide nucléique choisie parmi le groupe consistant en :
i) une molécule ADN consistant en une séquence tels que représenté sur la figure 2 ;
ii) une molécule ADN qui hybride dans des conditions d'hybridation contraignantes, à une séquence acide nucléique de la figure 2 et qui encode un inhibiteur d'activité apoptotique p53 ; et
iii) une molécule ADN qui est dégénérée en résultant d'un code génétique à des séquences ADN définies dans (a) et (b) ;
tandis que ladite étude comporte les étapes de :
a) fournir un échantillon de tissu isolé destiné à être testé ;
b) fournir des conditions adaptées pour la détection du Polypeptide par un agent et détecter la présence du polypeptide par l'agent ; et
c) comparer la régulation du polypeptide à des contrôles normaux en concordance.

2. Evaluation selon la revendication 1, **caractérisé en ce que** l'échantillon de tissu est un tissu de sein.

3. Evaluation selon la revendication 1 ou 2, **caractérisé en ce que** l'agent est un anticorps.

4. Evaluation selon la revendication 3, **caractérisé en ce qu'**elle est constituée d'une évaluation immunogénique (immunoassay).

5. Evaluation pour le diagnostic de cancer, **caractérisée par** une régulation d'une molécule acide nucléique dans un échantillon de tissu de poitrine isolé, lequel acide nucléique encode un inhibiteur de l'activité apoptotique, tandis que ladite molécule est choisie parmi le groupe consistant en :
i) une molécule acide nucléique consistant en une séquence telle que représentée par la figure 2 ;
ii) une molécule acide nucléique qui hybride la séquence acide nucléique dans la figure 2 et qui encoda un inhibiteur de l'activité apoptotique p53 ; et
iii) une molécule acide nucléique qui est dégénérée en résultant d'un code génétique aux séquences définies dans (a) et (b) ;
tandis que ladite évaluation comporte les étapes de :
a) fournir un échantillon destiné à être testé ;
b) fournir des conditions aptes à la détection de ladite molécule acide nucléique par ledit agent et détecter la présence de la molécule acide nucléique par l'agent ; et
c) comparer la régulation d'un acide nucléique à des contrôles normaux en concordance.

6. Evaluation selon la revendication 5, **caractérisée en ce que** l'agent est une molécule acide nucléique capable d'hybrider ladite séquence acide nucléique.

7. Evaluation selon la revendication 6, **caractérisée en ce que** l'acide nucléique est au moins un oligonucléotide.

8. Evaluation selon l'une des revendications 6 ou 7, **caractérisée en ce qu'**elle est une évaluation de réaction à chaîne polymérase.

9. Utilisation d'un anticorps apte à se lier à un polypeptide tel que représenté par la séquence acides aminés de la figure 3, pour la fabrication d'un médicament destiné à l'utilisation dans le traitement de cancer par induction d'apoptose.

10. Utilisation d'un anticorps selon la revendication 9, **caractérisée en ce que** le cancer est un cancer du sein.

11. Utilisation d'une molécule acide nucléique antisens comprenant une séquence anti sens d'une séquence sens choisie parmi le groupe consistant en :
i) une molécule acide nucléique consistant en une séquence telle que représentée sur la figure 2 ;
ii) une molécule acide nucléique consistant en une séquence qui hybride à la séquence acide nucléique de la figure 2 ; et
iii) une molécule acide nucléique qui consiste en une séquence qui est dégénérée en résultant du code génétique des séquences acide nucléique définies dans (a) et (b), pour l'utilisation dans la fabrication d'un médicament en vue du traitement du cancer par induction d'apoptose.

12. Procédé de balayage pour l'identification d'agents pharmacologiques qui interfèrent avec que la liaison d'un Polypeptide avec p53, comprenant :
i) fournir un polypeptide encodé par une molécule acide nucléique choisie parmi le groupe consistant en :
a) une molécule ADN consistant en une séquence tels que représentée par larnes séquences acide nucléique de la figure 2 ;
b) une molécule ADN qui hybride en la séquence acide nucléique de la figure 2 et qui encode un inhibiteur d'activité apoptotique p53 ; et
c) une molécule ADN qui est dégénérée en résultant d'un code génétique des séquences ADN définies dans i) et ii) ;
ii) fournir au moins un agent candidat ;
iii) fournir une préparation formant une combinaison de (i) et (ii) ;
iv) détecter ou mesuré l'activité de l'agent par rapport à l'interférence avec que la liaison dudit Polypeptide à p53 ; et de manière optionnelle
v) tester l'effet de l'agent sur l'apoptose d'un type de cellules choisies.

13. Procédé selon la revendication 12, **caractérisé en ce que** ledit polypeptide est encodé par la séquence acide nucléique représenté sur la figure 2.

14. Procédé selon la revendication 12 ou 13, **caractérisé en ce que** ledit polypeptide est exprimé par une cellule.

15. Procédé selon la revendication 14, **caractérisé en ce que** ladite cellules est une cellule de cancer.

16. Procédé selon l'une des revendications 12 à 15, **caractérisé en ce que** ledit polypeptide est surexprimé.

17. Procédé selon l'une des revendications 14 à 16, **caractérisé en ce que** la cellule est transformée/transfectée avec une molécule acide nucléique encodant ledit polypeptide.

18. Préparation pharmaceutique combinée comprenant au moins une molécule acide nucléique anti sens de séquence sens choisie parmi le groupe consistant en:
i) une molécule acide nucléique consistant en une séquence représenté sur la figure 2 ;
ii) une molécule acide nucléique consistant en une séquence qui hybride en la séquence de la figure 2 ; et
iii) une molécule acide nucléique qui contient une séquence qui est dégénérée en résultant d'un code génétique des séquences ADN définies dans (i) et (ii) et un agent chemo thérapeutique.

19. Préparation selon la revendication 18, **caractérisée en ce que** ledit agent chemo thérapeutique est un agent anticancer choisi parmi le groupe consistant en: cisplatine ; carboplatine ; cyclosphosphamide ; melphalan ; carmusline ; méthotrexate ; 5-fluorouracil ; cytarabine ; mercaptopurine ; daunorubicine ;doxorubicine ; épirubicine ; vinblastine ; vincristine ; dactinomycine ; mitomycine C ; taxale ; L-asparaginase ; G-CSF ; un énédiyne tel que chalichéamicine ou espéramicine ; chlorambucile ; ARA-C ; vindésine ; bléomycine ; ou étoposide.

20. Préparation selon la revendication 19, **caractérisée en ce que** ledit agent est cisplatine.

21. Préparation pharmaceutique combinée comprenant un anticorps capable de se lier à un polypeptide encodé par une molécule acide nucléique choisie parmi le groupe consistant en :
i) une molécule acide nucléique consistant en une séquence de la figure 2 ;
ii) une molécule acide nucléique consistant en une séquence qui hybride à une séquence acide nucléique de la figure 2 ; et
iii) une molécule acide nucléique qui consiste en une séquence qui est dégénérée en résultant d'un code génétique des séquences ADN définies dans (i) et (ii) et un agent chemo thérapeutique.

22. Préparation selon la revendication 21, **caractérisée en ce que** ledit agent chemo thérapeutique est choisi parmi le groupe consistant en : cisplatine ; carbopiatine ; eyctosphosphamide ; melphalan ; carmusline ; méthotrexate ; 5-fluorouracile ; cytarabine ; mercaptopurine ; daunorubicine ;doxorubicine ; épirubicine ; vinblastine ; vincristine ; dactinomycine ; mitomycine C ; taxole ; L-asparaginase ; 3-CSF ; un énédiyne tel que chalichéamicine ou espéramicine ; chlorambueile ; ARA-C ; vindésine ; bléomycine ; ou étoposide.

23. Préparation selon la revendication 22, **caractérisé en ce que** ledit agent chemo thérapeutique est cispiatine.
